# EUROPEAN PATENT SPECIFICATION

(11) **EP 4 326 162 B1**
(45) Date of publication and mention of the grant of the patent: **10.09.2025**
(21) Application number: 22723598.3
(22) Date of filing: 19.04.2022
(51) Int. Cl.: A61B 10/00

(54) **COMPUTING A POINT IN TIME FOR A FERTILITY MEDICAL ACTION**
BERECHNUNG EINES ZEITPUNKTS FÜR EINE MEDIZINISCHE FERTILITÄTSAKTION
CALCUL D'UN POINT DANS LE TEMPS POUR UNE ACTION MÉDICALE DE FERTILITÉ

(30) Priority: 22.04.2021 EP 21169895
(43) Date of publication of application: 28.02.2024
(73) Proprietor: De Belloy de Saint Liénard, Claire Marie Elisabeth, 6300 Zug (CH)
(72) Inventor: De Belloy de Saint Liénard, Claire Marie Elisabeth, 6300 Zug (CH)
(74) Representative: Peterreins Schley
(86) International application number: PCT/EP2022/060272
(87) International publication number: WO 2022/223535

(56) References cited:
- WO-A1-2021/034412
- KR-B1- 102 008 843
- US-A1- 2018 321 251
- US-A1- 2020 141 953

## Description

### TECHNICAL FIELD

This specification relates to a computer-implemented method for computing a point in time for a fertility medical action in a menstrual cycle of a female, and to a server in a network for computing the point in time for the fertility medical action and a portable analysis kit.

### BACKGROUND

Infertility can be seen as the inability of a person or animal to reproduce by natural means (with another person or another animal, respectively). In 2009, and in case of human beings, infertility has been recognized by the World Health Organization (WHO) as a disease, wherein infertility has been defined in the glossary by the International Committee for Monitoring Assisted Reproductive Technology (ICMART) and the WHO, https://www.who.int/reproductivehealth/publications/infertility/art_terminology2.pdf as "the failure to achieve a clinical pregnancy after 12 months or more of regular unprotected sexual intercourse". Various estimates on how many (heterosexual) couples are affected by infertility exist. As an example, the WHO states, as of September 14, 2020, that "estimates suggest that between 48 million couples and 186 million individuals live with infertility globally", https://www.who.int/news-room/fact-sheets/detail/infertility.

Infertility can be due to male infertility, to female infertility or to both male and female infertility. Male infertility may relate to a malfunction of the male reproductive system and "is most commonly caused by problems in the ejection of semen, absence or low levels of sperm, or abnormal shape (morphology) and movement (motility) of the sperm". Female infertility may relate to a malfunction of the female reproductive system and "may be caused by a range of abnormalities of the ovaries, uterus, fallopian tubes, and the endocrine system, among others". Again, estimates on the distribution of infertility causes vary. As a ballpark example, in about 30% of infertility cases, there is an issue only with the male, whereas in about 30% of infertility cases, there is an issue only with the female. In another 30 % of infertility cases, there is an issue with both the male and the female. On the other hand, in some cases (e.g. 10%) the infertility cause cannot be explained and/or no infertility cause is found.

In case of no issue with the male and no tubal factors with the woman, a usual first treatment of infertility may be timed sexual intercourse. The timing can be based on monitoring the natural cycle of the women and the injection of an oocyte maturation trigger in order to ensure proper ovulation. In case of human beings, such a trigger stimulus can e.g. comprise a human chorionic gonadotropin (hCG) trigger injection. Other trigger stimuli for inducing oocyte maturation may be gonadotropin-releasing hormone agonist GnRHa, both GnRHa and hCG administered in combination, recombinant luteinizing hormone (LH), and/or kisspeptin. The couple may then be advised to perform sexual intercourse at a specific point in time after this oocyte maturation injection. If these procedures are unsuccessful, another known (in)fertility treatment is an artificial insemination, wherein sperm is deliberately introduced into a female's cervix (i.e. intracervical insemination) or uterine cavity (i.e. intrauterine insemination) at a very precise time for the purpose of achieving pregnancy through in-vivo fertilization. The monitoring of these natural or modified natural cycles may often require several medical appointments in order to determine the right point in time for the oocyte maturation trigger injection, especially for women with irregular cycle. Even on women with regular cycles, the optimum point in time can change from cycle to cycle. These multiple appointments can be stressful for women who are working/living far away from a fertility clinic, as they may not be willing to reveal to their employer that they are trying to conceive.

In some cases, the infertility issue can be overcome by assisted reproductive technology (ART) used for (in)fertility treatment. As an example, if a fertility issue arises (only) from the male, e.g. from low sperm motility - and this issue cannot be resolved otherwise, e.g. through lifestyle measures or medication - infertility can be remedied by an in-vitro fertilization (IVF). The same applies, as another example, to infertility resulting from blocked fallopian tubes in the female reproductive system. IVF is a medical procedure where an egg (oocyte) is retrieved from the female's ovaries to be fertilized by the sperm outside the female body ("in a glass"). The procedure may involve monitoring and stimulating the ovulatory process of the female. In order to maximize chances of success, in conventional or stimulated IVF a female is to take large amounts of hormones in order to stimulate her/its ovaries, and produce multiple oocytes, instead of producing just one naturally. However, stimulation can be accompanied by side effects such as e.g. mood swings, weight gain, headache... on top of the stress of e.g. making multiple injections per day. Furthermore, another risk of the procedure is e.g. the ovarian hyperstimulation syndrome, which requires to stop the procedure (thereby delaying reproduction) and in some cases could be fatal. Still, conventional or stimulated IVF has an average success rate of e.g. 20-35% per cycle. Within conventional or stimulated IVF, the embryos which are not transferred as a fresh transfer can be frozen and transferred in another cycle, which is called frozen embryo transfer (FET). These FET can be done on an artificial cycle which is easy to monitor and plan or on a natural cycle which is more difficult to monitor, as per the same reasons of time sexual intercourse, an artificial insemination or a NC-IVF (explained hereafter). As an example, in an artificial cycle estrogen may be administered to stop natural ovulation and subsequently progesterone may be given.

An alternative to conventional or stimulated IVF (i.e. another (in)fertility treatment) is natural cycle in-vitro fertilization (NC-IVF) or modified natural cycle in-vitro fertilization. This procedure aims at respecting the natural menstrual cycle of the female in that NC-IVF does without hormone stimulation or keeps hormone stimulation to a minimum. It is therefore more bearable - in physiological and/or psychological terms - for the female to undergo NC-IVF, as it does not have the aforementioned side effects. Furthermore, in comparison to conventional or stimulated IVF, NC-IVF is usually less expensive per cycle (on average one third) but requires more cycles than conventional or stimulated IVF to achieve the same live birth rates (on average 2 to 3 cycles). Furthermore, NC-IVF by avoiding excess oocytes can be interesting for couples wishing to avoid embryo selection and/or embryo cryopreservation for personal and/or religious beliefs. NC-IVF is usually recommended for a (young) female in certain cases of male infertility, in case of blocked fallopian tubes, and/or for a female who has not responded well to hormone stimulation, for example due to a low ovarian reserve. NC-IVF is also an option for a female unsuccessful with conventional or stimulated IVF (e.g. who have not achieved a pregnancy).

According to the International Society for Mild Approaches in Assisted Reproduction (ISMAAR) NC-IVF is defined as an IVF without any medication. In this case, the oocyte of the female matures naturally, i.e. without any artificial hormone intake. On the other hand, NC-IVF (then sometimes also referred to as modified NC-IVF) may also include some medication such as, for example, an oocyte maturation trigger stimulus to induce final oocyte maturation before the oocyte retrieval. In case of human beings, such a trigger stimulus can e.g. comprise a human chorionic gonadotropin (hCG) trigger injection. Other trigger stimuli for inducing oocyte maturation may be gonadotropin-releasing hormone agonist GnRHa, both GnRHa and hCG administered in combination, recombinant luteinizing hormone (LH), and/or kisspeptin. Depending on the circumstances, medication may also comprise gonadotropin-releasing hormone (GnRH) antagonists (GnRHant) with or without follicle stimulation hormone (FSH) or human menopausal gonadotropin (hMG). Furthermore, a non-steroidal anti-inflammatory drug (NSAIDs) can be administered if the follicle is growing too fast as it can delay both its breakage and the release of the oocyte by a few hours. Also, low dosages of clomifene citrate (CC) may be used to avoid premature luteinizing hormone (LH) surge.

(Modified) NC-IVF can be problematic in that it can be more difficult for doctors to control the oocyte production and maturation. After all, the main challenge is to choose the right timing for the oocyte retrieval. In order to do so, it is essential to adapt the schedule of the procedure to the menstrual cycle of the female and to predict the optimal day so that the oocyte is mature enough but had not yet been released after/via follicle breakage. In fact, it has been shown that immature oocytes are less likely to be fertilized thereby reducing the probability of live pregnancy. Often, the size of a follicle measured in an ultrasound examination is a good indicator of the oocyte maturity given that the follicle is known to grow at a rate of approximately 2 mm per day and that a size close to e.g. 20 mm means that the follicle might break soon and release the oocyte. Thus a size reaching or exceeding 16 mm is deemed to indicate a right timing to plan the oocyte maturation trigger in order to achieve a state of optimal maturation e.g. 2 days later for the oocyte retrieval. On the other hand, such a measure could not always be precise due to the difficulty of measuring a non-perfect sphere in a 2D ultrasound examination. Another indication is known to result from a measure of estradiol (E2) (also named 17β-estradiol or oestradiol) exceeding certain thresholds in the menstrual cycle of the female, while a measure of luteinizing hormone (LH) exceeding certain thresholds in the menstrual cycle of the female may indicate a coming premature ovulation (in around 20 to 30% of cases). As an example, the oocyte maturation trigger injection (e.g. 5000 or 10000 international units/IU of human chorionic gonadotropin) should be administered (subcutaneously) when the follicle diameter has reached at least 16 mm and estradiol (E2) concentration has reached at least 700 pmol/L (or 734 pmol/L). In this case, typically, the oocyte retrieval should take place 36 hours after the trigger injection. As an example, precision of hormone measures (e.g. E2, LH) is required to be ± 100 pmol/L in order for them to function as reliable indicators.

There is a need to be able to monitor more often with less stress the women menstrual cycle (so at home) precisely enough in order to recommend the optimum point in time for e.g. the oocyte maturation trigger injection needed for several fertility medical actions, while helping preventing cycle cancellations due to premature ovulation. The objective is to increase the probability of pregnancies and live births of these fertility medical actions.

US 2020/141953 A1 discloses methods, for use in association with specially configured devices, systems, and kits, for performing immunoassay tests to detect for at least progesterone or analytes of progesterone on a sample in association with diagnosing problems and issues associated with corpus luteum functionality. The immunoassay devices and methods may be used in conjunction with diagnostic reader systems and/or a base unit for obtaining a sensitive read-out of the immunoassay results. The immunoassay devices and methods may utilize a competitive binding-like assay and a sandwich binding assay to detect at least progesterone or analytes of progesterone in a sample.

US 2018/321251 A1 discloses an in-home diagnostic system used to monitor ovulation cycles. In embodiments, the invention allows for users to evaluate early pregnancy status without medical training. Embodiments of the invention comprise a method incorporating usage of a smart device to allow for analysis of multiple analytes present within a single sample placed upon a lateral flow assay test cassette.

KR 102008843 B1 relates to a childbearing age test system. More specifically, the present invention relates to a smartphone-based childbearing age test system using saliva, which comprises the steps of: photographing a color change of childbearing age test paper according to the salinity of saliva with a wireless childbearing age tester; transmitting the photographed image data to a cloud server; determining the childbearing age by measuring an RGB value of the transmitted image data and comparing the measured RGB value with big data stored in the cloud server; and transmitting the result and related information to a smartphone to accurately provide the childbearing age status and related information in real time.

WO 2021/034412 A1 relates to a system and methods for evaluating and tracking the operation of the menstrual cycle and treating undesirable trends associated with the menstrual cycle. Various aspects of the system and methods described herein rely upon the operation of diagnostic tests specially configured to evaluate a bodily fluid for the presence or absence of hormones or analytes, and more specifically configured to evaluate a bodily fluid for at least the presence or absence of pregnanediol glucuronide at a threshold selected from the range inclusive of 1 µg/mL-10 µg/mL. The results from one or more diagnostic tests are interpreted in accordance with the teachings of the system. The interpretations are useful in accordance with facilitating treatments associated with medical conditions correlated to the generated interpretations, optionally delivered during a consultation with a medical provider during a telemedicine consultation, the treatments optionally comprising dietary changes incorporating the consumption of certain seeds to mitigate hormonal imbalances.

### SUMMARY

The invention is defined in claims 1, 13, and 14.

Dependent embodiments are given in the dependent claims and explained in the following description, to which the reader should now refer.

A fertility medical action in the menstrual cycle of the female may be an (in)fertility treatment (e.g., as discussed in the background, an oocyte maturation trigger stimulus, an oocyte retrieval for in-vitro fertilization, an embryo transfer, or an in-vivo insemination,...) or a timed sexual intercourse. The menstrual cycle of the female can be a natural cycle, a mono-ovulatory cycle, a modified natural cycle (e.g. as in modified natural cycle in-vitro fertilization), a stimulated cycle (e.g. as in conventional or stimulated in-vitro fertilization), or an artificial cycle (e.g. for embryo transfer). The point in time for the fertility medical action is computed to increase the probability of pregnancy for the female and/or of live birth after the fertility medical action. Whatever the fertility medical action (and the menstrual cycle), being able to precisely compute the point in time amounts to being able to compute an optimal point in time for the fertility medical action (or even of a sequence of fertility medical actions). Such is crucial for increasing the probability of pregnancy and/or of live birth thereby overcoming certain infertility causes (see e.g. background).

The method of claim 1 (or an embodiment thereof), the server of claim 13 (or an embodiment thereof) in the network, and the portable analysis kit of claim 14 (or an embodiment thereof) provide an easy-to-use (home/portable) solution assisting females (and, to some extent, corresponding males) in monitoring the menstrual cycle of the female and, eventually, in scheduling the fertility medical action. In so doing, appointments for (professional) cycle monitoring in a fertility clinic can be reduced to a minimum or avoided altogether. Along the same lines, thanks to the easy-to-use (home/portable) solution monitoring can be repeated at whatever monitoring frequency is desired. In so doing, the precision of the cycle monitoring and of the computed point in time for the fertility medical action can be increased, thereby increasing the probability of pregnancy and/or of live birth. Furthermore, the easy-to-use (home/portable) solution - while not restricted to fertility medical actions in a natural menstrual cycle of the female - particularly supports fertility medical actions in the natural menstrual cycle of the female, thereby reducing (conventionally encountered) cancellation and/or imprecision in the natural menstrual cycle of the female and, hence, increasing success rates for fertility medical actions in the natural menstrual cycle of the female. A conventional method to monitor the menstrual cycle of the female (here: of the woman) and to schedule the fertility medical action can be based on ultrasound examinations of the female ovaries in certain time intervals. As discussed in the background and as examples, follicles grow at a rate of approximately 2 mm per day and a follicle reaching or exceeding a size of 16 mm is deemed to be ready to plan the oocyte maturation trigger stimulus e.g. for oocyte retrieval for in-vitro fertilization. Ultrasound examinations are typically done by doctors and/or gynecologists. Another or an additional conventional method to monitor the menstrual cycle of the female comprises analyzing hormone levels of the female's blood in laboratory tests. Again, such testing requires professional assistance e.g. in a medical/fertility center. However, such professional examination and/or assistance is not always (and/or not everywhere) available for females. Furthermore, in certain phases of a fertility schedule it would be desirable to monitor the menstrual cycle more frequently, e.g. on a daily basis (for optimal adjustment to the menstrual cycle of the female thereby again increasing the probability of pregnancy and/or live birth). For obvious reasons, seeking professional examination/assistance e.g. on a daily basis puts a burden on females even in countries with highly developed health systems. In fact, many women undergoing a fertility schedule are healthy and most of them are actively engaged into working life. For female patients, especially for those coming from far away, going for e.g. an ultrasound examination is accompanied by an absence from work and additional unnecessary stress. Furthermore, for some women (and men) infertility is a delicate issue best kept private. In fact, even couples not suffering from infertility often wait to announce a pregnancy until having successfully passed a first critical stage (attributed to a higher risk of miscarriage) e.g. waiting to announce the pregnancy at least until the end of the first trimester, i.e. 12 weeks into the pregnancy. Sometimes the same applies to being on a fertility schedule where even future pregnancy (let alone live birth) is not guaranteed. Furthermore, some women prefer not to reveal their being on a fertility schedule (like their being pregnant) to their employers or associates for fear of (immediate) negative consequences such as being made redundant and/or being fired (depending on the jurisdiction). Psychological stress on females is known to have a negative impact on fertility and/or conception. It is therefore important to find means to reduce stress, in particular, in order to avoid worsening symptoms such as anxiety and/or depression. Thus, conventionally - and also to keep costs to a minimum (whoever has to bear them) - monitoring of the menstrual cycle of the female comprises but one or two checks per menstrual cycle before a fertility medical action such as e.g. an oocyte retrieval. In contrast, preferably (but not necessarily) in addition to (conventional) professional examination and/or assistance the easy-to-use (home/portable) solution provided by this specification allows the female to monitor the menstrual cycle of her own and at whatever monitor frequency is desired thereby keeping stress and expenditure(s) to a minimum. Chances of pregnancy and/or live birth are thereby increased.

Another problem that this specification is able to solve is related to the fact that menstrual cycles of females are not the same from cycle to cycle, from female to female, even with so-called regular females with no problems. As an example, due to such irregularities, a precocious oocyte can be easily missed, or slowly maturing oocytes can be retrieved too early. In this example, the optimal point in time of oocyte retrieval is known to vary from one cycle to another by up to several days. While, in general, doctors and/or gynecologists are skilled and experienced, a prediction of a point in time for a fertility medical action is often merely based on one measure of hormone which is compared to a small set of general pre-existing hormone curves (and the aforementioned less frequent examinations) and to a general growth curve of follicles. In contrast, the solution as disclosed in this specification can enable the female to input one or more items of meta data to account for e.g. age, body mass index, ethnic group, pre-conditions, anxiety status, weather data, lunar phase, such data (or one or more images of a blood or saliva sample) from the past, .... Such meta data (in addition to the at least one image of the blood or saliva sample) can be used to find a tailored prediction for the female based on predetermined sets of data from further females. On the other hand, such data (in addition to data on whether or not the fertility medical action for the further females proved successful) can be used to retrain the algorithm configured to compute the point in time of the fertility medical action. Put differently, thanks to successive (supervised) machine learning - and in contrast to the small set of general pre-existing hormone curves being used conventionally - the algorithm can automatically adapt to account for various groups of females and/or cycles. The computation of the point in time of the fertility medical action and the learning can advantageously be implemented on the server of the second aspect (or an embodiment thereof).

Conventionally, doctors/gynecologists determine the right point in time for a fertility medical action such as e.g. an oocyte maturation trigger injection/stimulus to a large extent on the follicle size. However, a measure of estradiol (E2) on the day of the oocyte maturation trigger injection/stimulus could be even more important to predict chances of pregnancy and/or live birth. Furthermore, it can also be advantageous to additionally consider a measure of luteinizing hormone (LH) in order to prevent premature ovulation on time. In fact, levels of estradiol (E2) and luteinizing hormone (LH) plotted against time in a menstrual cycle of a female may peak at different points in time. The difficulty of the exercise is to delay the oocyte retrieval as much as possible in order to have a more mature oocyte. On the other hand, the oocyte retrieval should not be after the follicle breakage as otherwise the oocyte cannot be retrieved and this would cancel the cycle. It can therefore be advantageous to also measure the luteinizing hormone (LH) because a surge of luteinizing hormone may indicate a state of premature ovulation that shall be avoided. Indeed, in case of an early LH rise, the oocyte maturation trigger stimulus needs to be given as soon as possible and the oocyte retrieval needs to be advanced as otherwise the ovulation might happen before the oocyte retrieval. In that case, a non-steroidal anti-inflammatory drug (NSAIDs) can be administered in order to delay the ovulation by a few hours.

The solution disclosed in this specification is particularly advantageous in case of a fertility medical action in a natural and/or modified natural menstrual cycle the female. In fact, in contrast to stimulated/or artificial menstrual cycles that are forced into synchronization with the fertility schedule, natural and/or modified natural menstrual cycles may be more irregular and thus harder to predict. However, even in case of (more) irregular (natural) menstrual cycles, the solution increases chances of pregnancy and/or live birth.

As already touched upon in the background, NC-IVF (and/or modified NC-IVF) provide the following advantages (as compared to conventional or stimulated IVF with hormone stimulation): Females do not suffer or suffer less from side effects and/or there is less risk of ovarian hyperstimulation syndrome. Furthermore, in case where just one oocyte is to be retrieved, full anesthesia is not mandatory as it can be replaced by pain killer medication. Furthermore, (modified) NC-IVF reduces psychological stress per cycle for the female and it can be e.g. 70% cheaper on average per cycle. Furthermore, it does not trigger the ethical dilemma of selecting and/or freezing (supernumerary) embryos. Furthermore, reducing the medications intake by the female (mother) reduces the risk of adverse perinatal outcomes that could come for example from supraphysiological estrogen concentrations in the blood circulation.

Additionally, the solution as disclosed in this specification can be used to optimize the point in time for one or more additional fertility medical actions (i.e. for a sequence of fertility medical actions). As an example, first a point in time for an oocyte maturation trigger stimulus can be computed, then a point in time for the oocyte retrieval.

Along the same lines the solution as disclosed in this specification can be used in an opposite sense for birth control e.g. in a natural menstrual cycle of the female.

### FIGURE DESCRIPTION

**Fig. 1a** schematically illustrates a computer-implemented method according to claim 1 (or an embodiment thereof) for computing a point in time for a fertility medical action in a menstrual cycle of a female.
**Fig. 1b** schematically illustrates an embodiment of a computer-implemented method according to claim 1 comprising determining a measure of estradiol in the blood or saliva sample.
**Fig. 1c** schematically illustrates an embodiment of a computer-implemented method according to claim 1 comprising determining a measure of estradiol and a measure of luteinizing hormone in the blood or saliva sample.
**Fig. 2a** schematically illustrates a server according to the claim 13 (or an embodiment thereof) and a portable analysis kit according to claim 14 (or an embodiment thereof).
**Fig. 2b** schematically illustrates a server according to claim 13 (or an embodiment thereof) and an embodiment of a portable analysis kit according to claim 14 comprising a portable analysis device with an image sensor.
**Fig. 2c** schematically illustrates a server according to claim 13 (or an embodiment thereof) and an embodiment of a portable analysis kit according to claim 14 comprising a client computer with an image sensor.
**Fig. 3a** illustrates an embodiment of an immunoassay strip comprising a first tine with a first testing zone.
**Fig. 3b** illustrates an embodiment of an immunoassay strip comprising a first tine with a first testing zone and a second testing zone.
**Fig. 3c** illustrates an embodiment of an immunoassay strip comprising a first tine with a first testing zone and a second tine with a second testing zone.
**Fig. 4** shows exemplary hormone curves in a menstrual cycle of a female human.

### DETAILED DESCRIPTION

The computer-implemented method, the server in a network, and the portable analysis kit provide an easy-to-use (home/portable) solution assisting females (and, to some extent, corresponding males) in monitoring the menstrual cycle of the female and, eventually, in scheduling the fertility medical action.

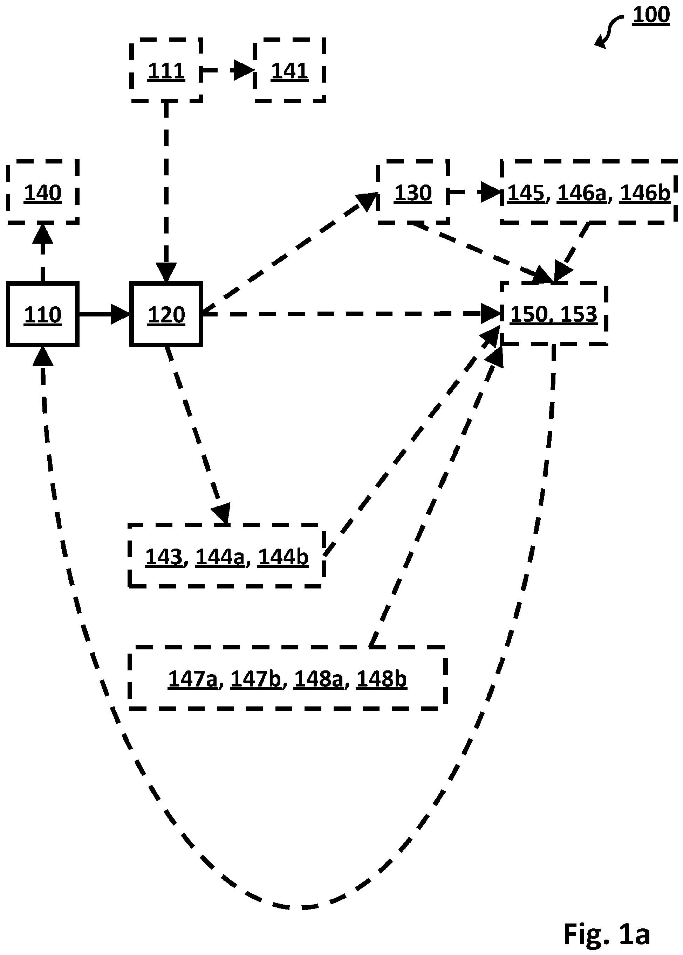

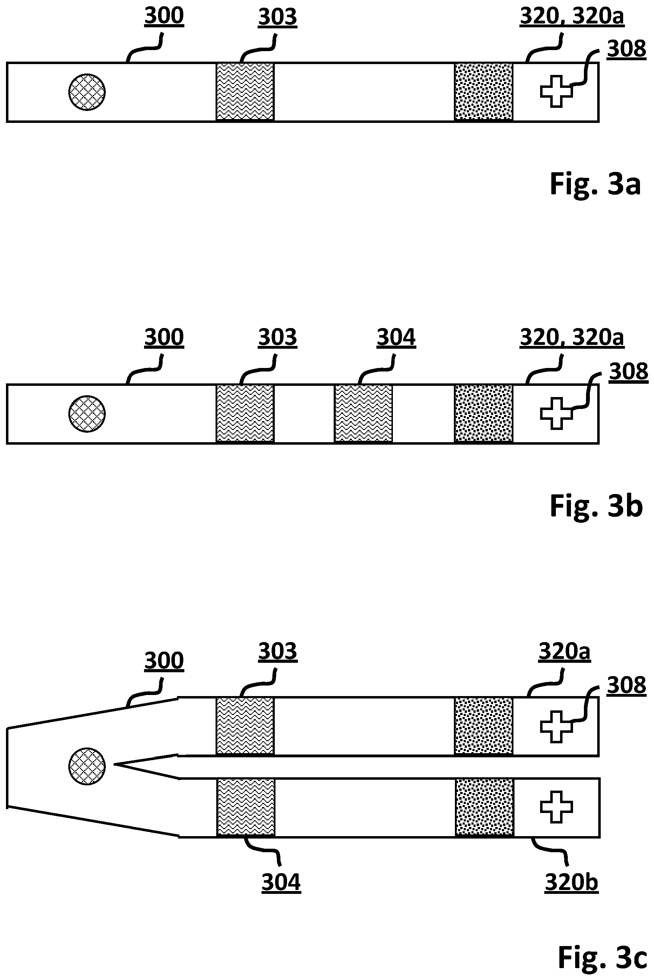

The computer-implemented method 100 for computing a point in time for a fertility medical action in a menstrual cycle of a female, comprises obtaining 110 at least one image of a blood or saliva sample processed on an immunoassay strip 300. As an example, the blood or saliva sample is first processed on the immunoassay strip 300 and subsequently the at least one image of the immunoassay strip 300 (or an appropriate portion thereof) is taken. On the other hand, the method 100 does not have to comprise taking/capturing the at least one image (as long as it is obtained, i.e. the at least one image may be taken elsewhere). The method further comprises computing 120 the point in time for the fertility medical action in the menstrual cycle of the female in part based on the at least one image of the blood or saliva sample processed on the immunoassay strip 300. Optionally, computing 120 the point in time for the fertility medical action in the menstrual cycle of the female is further based on predetermined sets of data from further females. The method is schematically illustrated in **Fig. 1a****(-c).** Exemplary embodiments of immunoassay strips are shown in **Fig. 3a****-c.**

The point in time for the fertility medical action can e.g. be characterized by a state of oocyte maturation (depending on the fertility medical action of interest). The point in time for the fertility medical action may be e.g. a timestamp specifying a day of the calendar and/or an hour of the day and/or a minute of the hour etc. in an appropriate timezone... The appropriate timezone may be user-defined or inferred otherwise (e.g. from settings of the client computer 220). Alternatively, the point in time for the fertility medical action can be just a day of the calendar, optionally specifying time intervals such as morning, afternoon, evening, and/or night (again depending on the fertility medical action of interest). Alternatively, instead of computing a point in time for the fertility medical action the method 100 can be adapted to computing a relative timespan from a current point in time (e.g. as of the blood or saliva sample).

The blood or saliva sample processed on the immunoassay strip 300 can be a blood sample processed on the immunoassay strip 300. Alternatively, it can be a saliva sample processed on the immunoassay strip 300. Instead of either a blood sample or a saliva sample processed on the immunoassay strip 300, the sample can be generalized throughout the specification to any body fluid processed on the immunoassay strip 300 provided that the body fluid contains at least one substance, e.g. at least one hormone, of the female that may serve as an indicator for a state in the menstrual cycle of the female. In fact, the body fluid sample can also be a serum sample processed on the immunoassay strip 300. Alternatively, but not claimed, the body fluid sample can be a urine sample processed on the immunoassay strip 300. Alternatively, but not claimed, the body fluid sample can be a vaginal mucus sample processed on the immunoassay strip 300. Furthermore, it is conceivable to combine two or more of the aforementioned samples (e.g. a blood sample and a saliva sample) and generalizing the method 100 to one or more images of the corresponding two or more samples processed on one or more immunoassay strips. The at least one image of the blood or saliva sample (or one of the aforementioned samples) may be a digital image such as e.g. a bitmap or a jpeg etc. The method 100 may be further generalized to comprise obtaining a sequence of images of the blood or saliva sample processed on the immunoassay strip 300 or of the two or more aforementioned samples.

The female (i.e. the one for whom the point in time for the fertility medical action is computed) may or may not be included in the further females. For each such further female at least one predetermined set of data is available comprising information about at least one image of a blood or saliva sample processed on an immunoassay strip, one or more items of meta data, a fertility schedule (e.g. a timespan between the blood or saliva sample and the fertility medical action), and/or a status on whether or not the further fertility medical action resulted in pregnancy of the further female (and/or live birth). As an example, the predetermined sets of data from the further females can be stored (in a database) on a server 160.

The point in time for the fertility medical action may be computed to increase the probability of pregnancy for the female and/or of live birth after the fertility medical action. The female can be a female human (i.e. a woman). Alternatively, the female can be a female animal (other than a woman). Animals of economic interest may e.g. be horses, cattle, domestic pigs, dogs, or cats.

The fertility medical action may be an oocyte maturation trigger stimulus, optionally wherein the oocyte maturation trigger stimulus induces final oocyte maturation. As an example, the maturation trigger stimulus can be an oocyte maturation trigger injection (to be administered intramuscularly or subcutaneously, e.g. a shot under the skin on the abdomen). In case of a woman, the substance to be injected as the oocyte maturation trigger may be e.g. human chorionic gonadotropin (e.g. 5000 IU or 10000 IU). On the other hand, the substance to be injected as the oocyte maturation trigger may be equine chorionic gonadotropin (for horses), bovine chorionic gonadotropin (for cattle) etc. Other trigger stimuli for inducing oocyte maturation may be gonadotropin-releasing hormone agonist GnRHa, both GnRHa and hCG administered in combination, recombinant luteinizing hormone (LH), and/or kisspeptin. Alternatively, or in addition (i.e. in a sequence of one or more fertility medical actions), the fertility medical action may be an oocyte retrieval, optionally wherein an oocyte retrieved in the oocyte retrieval is used for in-vitro fertilization (e.g. conventional or stimulated in-vitro fertilization IVF, modified or natural cycle in-vitro fertilization (NC-IVF). Alternatively, or in addition (i.e. in a sequence of one or more fertility medical actions), the fertility medical action may be a transfer of the fertilized egg (zygote/embryo) into the female's fallopian tubes. Alternatively, or in addition (e.g. in a sequence of one or more fertility medical actions), the fertility medical action may be a transfer of the embryo into the female's uterus. Alternatively, the fertility medical action may be an embryo transfer (e.g. a frozen embryo transfer) e.g. during another cycle of the women. Alternatively, the fertility medical action may be an in-vivo insemination such as e.g. an insemination into a female's cervix (i.e. intracervical insemination) or uterine cavity (i.e. intrauterine insemination). Alternatively, the in-vivo insemination may be an intravaginal insemination or an intratubal insemination. Alternatively, the fertility medical action may be a timed sexual intercourse. An oocyte retrieval (and, hence, an in-vitro fertilization and/or a transfer of the fertilized egg), an embryo transfer, an in-vivo insemination, and a timed sexual intercourse may be preceded by the oocyte maturation trigger stimulus.

The menstrual cycle of the female may be a natural cycle. Alternatively, the menstrual cycle of the female may be a mono-ovulatory cycle. Alternatively, the menstrual cycle of the female may be a modified natural cycle. Alternatively, the menstrual cycle of the female may be a stimulated cycle. Alternatively, the menstrual cycle of the female may be an artificial cycle.

Obtaining 110 the at least one image of the blood or saliva sample processed on the immunoassay strip 300 may comprise obtaining (i.e. taking/capturing) the at least one image of the blood or saliva sample processed on the immunoassay strip 300 via at least one image sensor 210 of a portable analysis kit 200. As an example, the at least one image of the blood or saliva sample may be taken by the at least one image sensor 210 of the portable analysis kit 200. In cases, where the portable analysis kit 200 comprises a portable analysis device 230 comprising the at least one image sensor 210, the at least one image of the blood or saliva sample may be transferred via at least one communication interface 233 of the portable analysis device 230. Alternatively, or in addition, obtaining 110 the at least one image of the blood or saliva sample processed on the immunoassay strip 300 may result from taking the at least one image of the blood or saliva sample processed on the immunoassay strip 300 using a smart device. Generally, a smart device may be an electronic device, generally connected to other devices or networks via different wireless protocols such as Bluetooth, Zigbee, NFC, Wi-Fi, LiFi, 4G, 5G, 6G, etc., that can operate to some extent interactively and autonomously. The smart device may be e.g. a smartphone, a tablet or the like... The at least one image of the blood or saliva sample processed on the immunoassay strip 300 may have a timestamp (specifying a day of the calendar and/or an hour of the day and/or a minute of the hour etc. e.g. in the aforementioned appropriate timezone). Alternatively, the at least one image can be deemed to be as of now when obtained 110.

For example, the smart device/portable analysis device 230 (e.g. a smartphone) comprising a digital camera 211 may be used as in https://kaloramainformation.com/blog/smartphones-and-ivd-early-but-getting-there and/or https://www.bbisolutions.com/en/digital/diagnostic-app-development.

Computing 120 the point in time for the fertility medical action in the menstrual cycle of the female in part based on the at least one image of the blood or saliva sample processed on the immunoassay strip 300 may comprise applying the at least one image to an image-correction algorithm configured to detect and to correct misalignment (if existing and/or if needed), wrong scaling (if existing and/or if needed) and/or distortions (if existing and/or if needed) of the immunoassay strip 300 in the at least one image of the blood or saliva sample, thereby updating the at least one image of the blood or saliva sample. Such an image-correction algorithm may be advantageous for smart device shots from various angles/distances or if the lateral flow immunochromatographic assay 300 is misaligned in a stage 231 of the portable analysis device 230. Such detection and correction may be based on e.g. at least one fiducial 308 (a line, a cross, etc. as shown in **Fig. 3a****-c**) on the lateral flow immunochromatographic assay 300 and/or via machine learning. On the other hand, some machine learning algorithms (here: the first or second machine learning algorithm discussed below) may not need such image-correction (if trained appropriately on such input). The stage 231 of the portable analysis device 230 - in particular, if the portable analysis device 230 further comprises the at least one image sensor 210 - may be configured so as to prevent the immunoassay strip 300 from misalignment, wrong scaling and/or distortions.

Computing 120 the point in time for the fertility medical action in the menstrual cycle of the female in part based on the at least one image of the blood or saliva sample processed on the immunoassay strip 300 may comprise applying the at least one image to a calibration algorithm configured to calibrate a colormap and/or a grayscale map of the at least one image based on at least one reference zone on the immunoassay strip 300 in the at least one image of the blood or saliva sample, thereby updating the at least one image of the blood or saliva sample. As an example, the at least one reference zone may feature one or more reference colors (potentially including gray tones, black, and/or white). The calibration algorithm can be advantageously used e.g. to account for varying lightning conditions.

Computing 120 the point in time for the fertility medical action in the menstrual cycle of the female in part based on the at least one image of the blood or saliva sample processed on the immunoassay strip 300 may comprise applying the at least one image of the blood or saliva sample to at least one digital filter (or a sequence of filters), thereby updating the at least one image of the blood or saliva sample. Alternatively, one or more analogous filters (also a sequence of such filters) may be used, e.g. in the portable analysis device 230. Regardless of whether the at least one filter is digital or analogous, it may be local (i.e. depend on the geometry of bands on the test strip. As an example, one local portion of the filter may be used for a (first) testing zone 303 for measuring estradiol (E2), whereas another local portion of the filter may be used for a (second) testing zone 304 for measuring luteinizing hormone (LH).

The at least one digital filter may comprise a grayscale filter and/or a monochromic filter for at least one set of pixels of the at least one image of the blood or saliva sample. Such may be advantageous if only an intensity and/or a binary result of testing zones 303, 304 on the immunoassay strip 300 matter. The at least one set of pixels may be a proper subset of the pixels or all pixels. Furthermore, several such subsets may correspond to (the) one or more testing zones 303, 304 on the immunoassay strip 300.

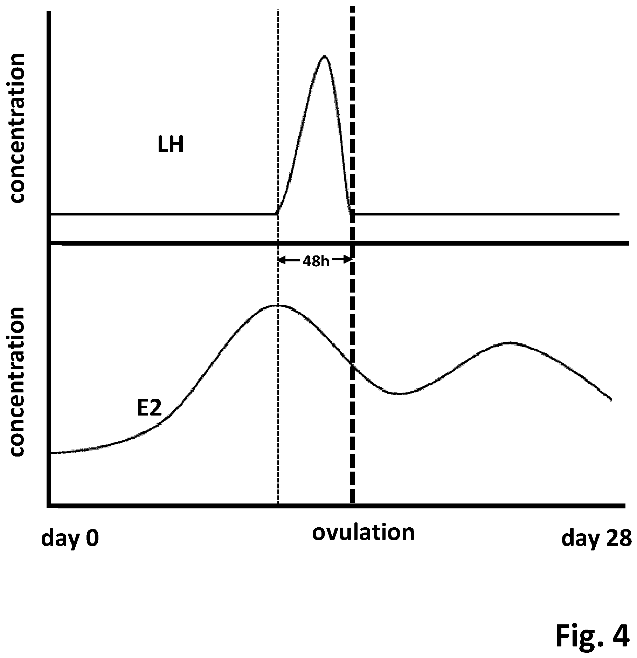

The method 100 may further comprise obtaining 111 one or more items of meta data, optionally via (a user interface of) a client computer 220 of a portable analysis kit 200. As discussed in the summary above, the one or more items of meta data can advantageously be used to compute a tailored point in time for the fertility medical action. In so doing, in comparison to conventionally using a small set of general pre-existing hormone curves, the precision of the optimal point in time for the fertility medical action can be increased. One item of meta data may be the age of the female. Alternatively, or in addition, one item (or another item) of meta data may be the body mass index of the female (or height and weight of the female). Alternatively, or in addition, one item (or another item) of meta data may be the ethnic group (or a mix of ethnic groups) of the female. Alternatively, or in addition, one item (or another item) of meta data may be one or more pre-conditions (e.g. the medical history) of the female. Alternatively, or in addition, one item (or another item) of meta data may be psychological data (e.g. concerning an anxiety status and/or levels of stress/depression) of the female. Alternatively, or in addition, one item (or another item) of meta data may be weather data (e.g. inside/outside temperature, air humidity, sun intensity, season) for the female. To some extent such weather data may be inferred from a geographic location and corresponding weather data queried from the internet. Alternatively, or in addition, one item (or another item) of meta data may be a geographic location (e.g. in terms of coordinates, a name of a location and/or of a country) of the female. Alternatively, or in addition, one item (or another item) of meta data may be astronomical data (e.g. a lunar phase). Such data may be inferred from the geographic location of the female and a timestamp. Alternatively, or in addition, one or more items may comprise any such data from the past. Alternatively, or in addition, one or more items may comprise one or more images of a blood or saliva sample of the female from the past. As an example, knowledge of previously timestamped images may advantageously be used to resolve ambiguities in hormone curves. As an example, and as illustrated in **Fig. 4****,** a single measure of estradiol (E2) is sometimes not enough to decide whether the current state (say, at around 300 pg/mL) of oocyte maturation is right before or right after the estradiol (E2) peak. However, another measure of e.g. luteinizing hormone (LH) may be advantageously used to prevent (the risk of) premature ovulation and thus cancellation of the cycle. Such may, in fact, be achieved by further postulating that the measure of luteinizing hormone (LH) must not be above a predetermined threshold. Alternatively, or in addition, one or more items may comprise data from one or more previous fertility medical actions and corresponding statuses on whether or not the one or more previous fertility medical action resulted in pregnancy of the further female (and/or live birth).

Computing 120 the point in time for the fertility medical action in the menstrual cycle of the female in part based on the at least one image of the blood or saliva sample processed on the immunoassay strip 300 comprises applying the at least one image of the blood or saliva sample to an analysis algorithm and, optionally, applying the one or more items of meta data to the analysis algorithm (and using them). The analysis algorithm comprises a machine learning algorithm. Machine learning is advantageously used to successively improve a model for computing 120 the point in time for the fertility medical action. The analysis algorithm comprises (or is) a first pre-trained machine learning algorithm configured to compute the point in time for the fertility medical action in the menstrual cycle of the female. As an example, the first pre-trained machine learning algorithm may be trained in supervised learning based on predetermined training data (e.g. on the predetermined sets of data from the further females) using machine learning techniques (such as e.g. stochastic gradient descent) to minimize a cost function on the training data. The first pre-trained machine learning algorithm may represent a regression and/or a classification. As an example, a regression can be used to compute/predict a (continuous) value such as a timespan to compute the point in time for the fertility medical action. Alternatively, or in addition, a predetermined discrete set of timespan intervals (here: the classes) can be used in a classification, again computing/predicting one of the classes of timespan intervals to compute the point in time for the fertility medical action. Alternatively, or in addition, a probability of confidence may be computed/predicted (e.g. via a regression, or via classification and a soft-max cost function). The first pre-trained machine learning algorithm may comprise (or be) an artificial neural network and/or a convolutional neural network. As an example, the first pre-trained machine learning algorithm may be a composition of one or more artificial neural networks and one or more convolutional neural networks. A convolutional neural network (e.g. as a part of the aforementioned composition) can be advantageously used to account for translational invariance of the at least one image of the blood or saliva sample to be analyzed. Depending on the number of hidden layers in the artificial neural networks and/or in the convolutional neural networks learning may be referred to as shallow or deep learning. The immunoassay strip 300 may be configured to test one hormone, e.g. estradiol (E2) or luteinizing hormone (LH). Alternatively, the immunoassay strip 300 may be configured to test two hormones, e.g. estradiol (E2) and luteinizing hormone (LH).

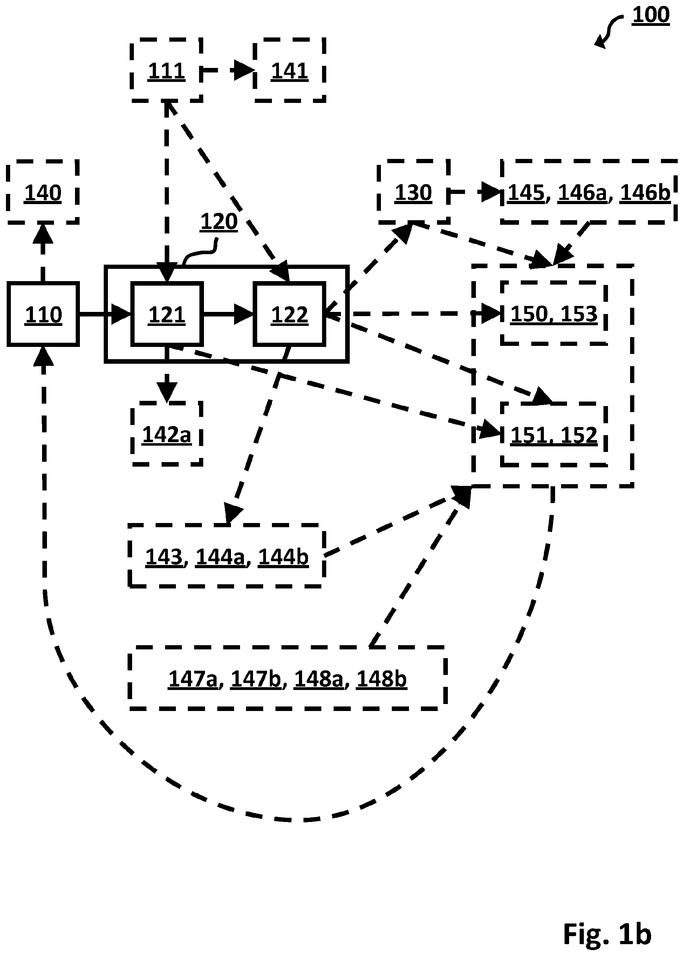

In an embodiment (embodiment determining estradiol), computing 120 the point in time for the fertility medical action in the menstrual cycle of the female in part based on the at least one image of the blood or saliva sample processed on the immunoassay strip 300 may comprise determining 121 a measure of estradiol (E2) in the blood or saliva sample based on the at least one image of the blood or saliva sample and computing 122 the point in time for the fertility medical action in the menstrual cycle of the female in part based on the measure of estradiol in the blood or saliva sample. Put differently, in this embodiment computing 120 the point in time for the fertility medical action divides into two subsequent steps, as e.g. illustrated in **Fig. 1b****.** In principle, estradiol (E2) could be replaced by another estrogen such as e.g. estrone (E1), estriol (E3), estetrol (E4), estrone-3-glucuronide (E3G), albeit that estetrol (E4) is only produced during pregnancy. On the other hand, estradiol (E2) may be a preferred estrogen as estradiol levels and changes thereof seem to function as good indicators corresponding to various states in the menstrual cycle of the female, see e.g. **Fig. 4****.** Alternatively, and in principle, estradiol (E2) may be replaced by e.g. luteinizing hormone (LH). The measure of estradiol (E2) may be a concentration correlated or equivalent to a chemical concentration (e.g. in pmol/L or pg/mL), i.e. to a number. The concentration may be correlated (e.g. be proportional) to the number of molecules of estradiol (E2) flowed into one or more testing zones. Alternatively, the measure of estradiol (E2) may be a concentration pattern given in terms of concentrations corresponding to one or more testing zones 303, 304 on the immunoassay strip 300. Alternatively, the measure of estradiol (E2) does not have to be an interpretable concentration, let alone a number. The concentration/measure of estradiol can be a multi-dimensional object. As an example, the multi-dimensional object may be a vector, a matrix, or a tensor. As an example, in case of an immunoassay strip with two testing zones for estradiol, the concentration may be given in terms of a vector of two numbers corresponding to the concentrations measured on the two testing zones. As another example, the measure of estradiol (E2) may be an auto-encoding surrogate (e.g. for image compression) of the at least one image of the blood or saliva sample processed on the immunoassay strip 300.

In this embodiment (embodiment determining estradiol), the analysis algorithm may comprise a sample-to-measure algorithm. Applying the at least one image of the blood or saliva sample to the analysis algorithm and, optionally, applying the one or more items of meta data to the analysis algorithm may then comprises applying the at least one image of the blood or saliva sample to the sample-to-measure algorithm and, optionally, applying the one or more items of meta data to the sample-to-measure algorithm. The sample-to-measure algorithm does not have to comprise a machine learning algorithm. On the other hand, machine learning may advantageously be used to successively improve a model for determining 121 the measure of estradiol in the blood or saliva sample based on the at least one image of the blood or saliva sample. The sample-to-measure algorithm may comprise (or be) a second pre-trained machine learning algorithm configured to determine 121 the measure of estradiol in the blood or saliva sample based on the at least one image of the blood or saliva sample. As an example, the second pre-trained machine learning algorithm may be trained in supervised learning based on predetermined training data (e.g. on the predetermined sets of data from the further females) using machine learning techniques (such as e.g. stochastic gradient descent) to minimize a cost function on the training data. The second pre-trained machine learning algorithm may represent a regression and/or a classification. As an example, a regression can be used to compute/predict a (continuous) value such as the measure of estradiol (E2) in terms of a number in a unit of concentration. Alternatively, or in addition, a predetermined discrete set of intervals in the unit of concentration (here: the classes) can be used in a classification, again computing/predicting one of the classes of intervals in the unit of concentration. Alternatively, or in addition, a probability of confidence may be computed/predicted (e.g. via a regression, or via classification and a soft-max cost function). The second pre-trained machine learning algorithm may comprise (or be) an artificial neural network and/or a convolutional neural network. As an example, the second pre-trained machine learning algorithm may be a composition of one or more artificial neural networks and one or more convolutional neural networks. A convolutional neural network (e.g. as a part of the aforementioned composition) can be advantageously used to account for translational invariance of the at least one image of the blood or saliva sample to be analyzed. Depending on the number of hidden layers in the artificial neural networks and/or in the convolutional neural networks learning may be referred to as shallow or deep learning. The second pre-trained machine learning algorithm may or may not be the first pre-trained machine learning algorithm (or a portion thereof).

In this embodiment (embodiment determining estradiol), the analysis algorithm may comprise a measure-to-point-in-time algorithm. Computing 122 the point in time for the fertility medical action in the menstrual cycle of the female in part based on the measure of estradiol in the blood or saliva sample may comprise applying the measure of estradiol in the blood or saliva sample to the measure-to-point-in-time algorithm and, optionally, applying the one or more items of meta data to the measure-to-point-in-time algorithm. The measure-to-point-in-time algorithm does not have to comprise a machine learning algorithm. On the other hand, machine learning may advantageously be used to successively improve a model for compute 122 the point in time for the fertility medical action in the menstrual cycle of the female in part based on the measure of estradiol in the blood or saliva sample. The measure-to-point-in-time algorithm may comprise (or be) a third pre-trained machine learning algorithm configured to compute 122 the point in time for the fertility medical action in the menstrual cycle of the female in part based on the measure of estradiol in the blood or saliva sample. As an example, the third pre-trained machine learning algorithm may be trained in supervised learning based on predetermined training data (e.g. on the predetermined sets of data from the further females, in particular, comprising corresponding measures of estradiol in the blood or saliva samples) using machine learning techniques (such as e.g. stochastic gradient descent) to minimize a cost function on the training data. The third pre-trained machine learning algorithm may represent a regression and/or a classification. As an example, a regression can be used to compute/predict a (continuous) value such as a timespan to compute the point in time for the fertility medical action. Alternatively, or in addition, a predetermined discrete set of timespan intervals (here: the classes) can be used in a classification, again computing/predicting one of the classes of timespan intervals to compute the point in time for the fertility medical action. Alternatively, or in addition, a probability of confidence may be computed/predicted (e.g. via a regression, or via classification and a soft-max cost function). The third pre-trained machine learning algorithm may comprise (or be) an artificial neural network and/or a convolutional neural network. As an example, the third pre-trained machine learning algorithm may be a composition of one or more artificial neural networks and one or more convolutional neural networks. Depending on the number of hidden layers in the artificial neural networks and/or in the convolutional neural networks learning may be referred to as shallow or deep learning. The third pre-trained machine learning algorithm may or may not be the first pre-trained machine learning algorithm (or a portion thereof). The first pre-trained machine learning algorithm may be a composition of the second pre-trained machine learning algorithm and (in the sense of subsequently) the third pre-trained machine learning algorithm.

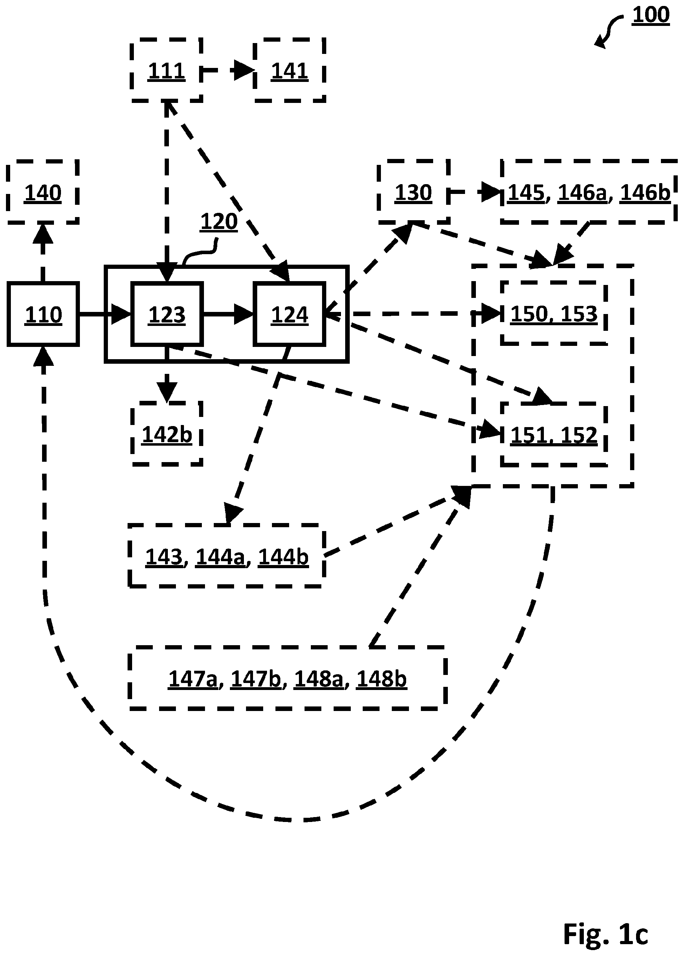

In an embodiment (embodiment determining estradiol and luteinizing hormone), computing 120 the point in time for the fertility medical action in the menstrual cycle of the female in part based on the at least one image of the blood or saliva sample processed on the immunoassay strip 300 may comprise determining 123 a measure of estradiol (E2) in the blood or saliva sample and a measure of luteinizing hormone (LH) in the blood or saliva sample based on the at least one image of the blood or saliva sample and computing 124 the point in time for the fertility medical action in the menstrual cycle of the female in part based on the measure of estradiol in the blood or saliva sample and on the measure of luteinizing hormone in the blood or saliva sample. Put differently, in this embodiment computing 120 the point in time for the fertility medical action divides into two subsequent steps, as e.g. illustrated in **Fig. 1c**. Again, in principle, estradiol (E2) could be replaced by another estrogen such as e.g. estrone (E1), estriol (E3), estetrol (E4), estrone-3-glucuronide (E3G), albeit that estetrol (E4) is only produced during pregnancy. On the other hand, estradiol (E2) may be a preferred estrogen as estradiol levels and changes thereof seem to function as good indicators corresponding to various states in the menstrual cycle of the female, see e.g. Fig. 4. The measure of estradiol (E2) may be a concentration correlated or equivalent to a chemical concentration (e.g. in pmol/L or pg/mL), i.e. to a number. The concentration may be correlated (e.g. be proportional) to the number of molecules of estradiol (E2) flowed into one or more testing zones. Alternatively, or in addition, the measure of luteinizing hormone (LH) may be a concentration correlated or equivalent to a chemical concentration (e.g. in pmol/L or pg/mL), i.e. to a number. The concentration may be correlated (e.g. be proportional) to the number of molecules of luteinizing hormone (LH) flowed into one or more testing zones. Alternatively, the measure of estradiol (E2) and/or the measure of luteinizing hormone (LH) may be a concentration pattern given in terms of concentrations corresponding to one or more testing zones 303, 304 on the immunoassay strip 300. Alternatively, the measure of estradiol (E2) does not have to be an interpretable concentration, let alone a number. The concentration/measure of estradiol can be a multi-dimensional object. As an example, the multi-dimensional object may be a vector, a matrix, or a tensor. As an example, in case of an immunoassay strip with two testing zones for estradiol, the concentration for estradiol (E2) may be given in terms of a vector of two numbers corresponding to the concentrations measured on the two testing zones. Alternatively, or in addition, the measure of luteinizing hormone (LH) does not have to be an interpretable concentration, let alone a number. The concentration/measure of luteinizing hormone can also be multi-dimensional object. As an example, the multi-dimensional object may be a vector, a matrix, or a tensor. As an example, in case of an immunoassay strip with two testing zones for luteinizing hormone, the concentration for luteinizing hormone (LH) may be given in terms of a vector of two numbers corresponding to the concentrations measured on the two testing zones. As another example, the measure of estradiol (E2) and/or the measure of luteinizing hormone (LH) may be an auto-encoding surrogate (e.g. for image compression) of the at least one image of the blood or saliva sample processed on the immunoassay strip 300.

In this embodiment (embodiment determining estradiol and luteinizing hormone), the analysis algorithm may comprise a sample-to-measure algorithm. Applying the at least one image of the blood or saliva sample to the analysis algorithm and, optionally, applying the one or more items of meta data to the analysis algorithm may comprise applying the at least one image of the blood or saliva sample to the sample-to-measure algorithm and, optionally, applying the one or more items of meta data to the sample-to-measure algorithm. The sample-to-measure algorithm does not have to comprise a machine learning algorithm. On the other hand, machine learning may advantageously be used to successively improve a model for determining 123 the measure of estradiol in the blood or saliva sample and the measure of luteinizing hormone in the blood or saliva sample based on the at least one image of the blood or saliva sample. The sample-to-measure algorithm may comprise (or be) a second pre-trained machine learning algorithm configured to determine 123 the measure of estradiol in the blood or saliva sample and the measure of luteinizing hormone in the blood or saliva sample based on the at least one image of the blood or saliva sample. As an example, the second pre-trained machine learning algorithm may be trained in supervised learning based on predetermined training data (e.g. on the predetermined sets of data from the further females) using machine learning techniques (such as e.g. stochastic gradient descent) to minimize a cost function on the training data. The second pre-trained machine learning algorithm may represent a regression and/or a classification. As an example, a regression can be used to compute/predict (continuous) values such as for the measure of estradiol (E2) and for the measure of luteinizing hormone (LH), each in terms of a number in a unit of concentration. Alternatively, or in addition, a predetermined discrete set of two-dimensional intervals in the unit of concentration (here: the classes) can be used in a classification, again computing/predicting one of the classes of two-dimensional intervals in the unit of concentration. Alternatively, or in addition, a probability of confidence may be computed/predicted (e.g. via a regression, or via classification and a soft-max cost function). The second pre-trained machine learning algorithm may comprise (or be) an artificial neural network and/or a convolutional neural network. As an example, the second pre-trained machine learning algorithm may be a composition of one or more artificial neural networks and one or more convolutional neural networks. A convolutional neural network (e.g. as a part of the aforementioned composition) can be advantageously used to account for translational invariance of the at least one image of the blood or saliva sample to be analyzed. Depending on the number of hidden layers in the artificial neural networks and/or in the convolutional neural networks learning may be referred to as shallow or deep learning. The second pre-trained machine learning algorithm may or may not be the first pre-trained machine learning algorithm (or a portion thereof).

In this embodiment (embodiment determining estradiol and luteinizing hormone), the analysis algorithm may comprise a measure-to-point-in-time algorithm. Computing 124 the point in time for the fertility medical action in the menstrual cycle of the female in part based on the measure of estradiol and on the measure of luteinizing hormone in the blood or saliva sample may comprise applying the measure of estradiol in the blood or saliva sample and the measure of luteinizing hormone in the blood or saliva sample to the measure-to-point-in-time algorithm and, optionally, applying the one or more items of meta data to the measure-to-point-in-time algorithm. The measure-to-point-in-time algorithm does not have to comprise a machine learning algorithm. On the other hand, machine learning may advantageously be used to successively improve a model for computing 124 the point in time for the fertility medical action in the menstrual cycle of the female in part based on the measure of estradiol in the blood or saliva sample and on the measure of luteinizing hormone in the blood or saliva sample. The measure-to-point-in-time algorithm may comprise (or be) a third pre-trained machine learning algorithm configured to compute 124 the point in time for the fertility medical action in the menstrual cycle of the female in part based on the measure of estradiol in the blood or saliva sample and on the measure of luteinizing hormone in the blood or saliva sample. As an example, the third pre-trained machine learning algorithm may be trained in supervised learning based on predetermined training data (e.g. on the predetermined sets of data from the further females, in particular, comprising corresponding measures of estradiol and luteinizing hormone in the blood or saliva samples) using machine learning techniques (such as e.g. stochastic gradient descent) to minimize a cost function on the training data. The third pre-trained machine learning algorithm may represent a regression and/or a classification. As an example, a regression can be used to compute/predict a (continuous) value such as a timespan to compute the point in time for the fertility medical action. Alternatively, or in addition, a predetermined discrete set of timespan intervals (here: the classes) can be used in a classification, again computing/predicting one of the classes of timespan intervals to compute the point in time for the fertility medical action. Alternatively, or in addition, a probability of confidence may be computed/predicted (e.g. via a regression, or via classification and a soft-max cost function). The third pre-trained machine learning algorithm may comprise (or be) an artificial neural network and/or a convolutional neural network. As an example, the third pre-trained machine learning algorithm may be a composition of one or more artificial neural networks and one or more convolutional neural networks. Depending on the number of hidden layers in the artificial neural networks and/or in the convolutional neural networks learning may be referred to as shallow or deep learning. The third pre-trained machine learning algorithm may or may not be the first pre-trained machine learning algorithm (or a portion thereof). The first pre-trained machine learning algorithm may be a composition of the second pre-trained machine learning algorithm and (in the sense of subsequently) the third pre-trained machine learning algorithm.

In general, the first pre-trained machine learning algorithm may have been trained in part based on the predetermined sets of data from further females, optionally wherein each of the predetermined sets of data comprises at least one further image of a further female's blood or saliva sample processed on an immunoassay strip, a (corresponding) timespan for a (corresponding) further fertility medical action indicating a temporal difference between the at least one further image and the (corresponding) further fertility medical action, and a (corresponding) status on whether or not the (corresponding) further fertility medical action resulted in (or contributed to) pregnancy of the (corresponding) further female and/or live birth. The second pre-trained machine learning algorithm may have been trained in part based on the predetermined sets of data from further females, optionally wherein each of the predetermined sets of data comprises at least one further image of a further female's blood or saliva sample processed on an immunoassay strip, a (corresponding) measure of estradiol and/or a (corresponding) measure of luteinizing hormone for the further female's blood or saliva sample. The third pre-trained machine learning algorithm may have been trained in part based on the predetermined sets of data from further females, optionally wherein each of the predetermined sets of data comprises a measure of estradiol and/or a measure of luteinizing hormone for a further female's blood or saliva sample processed on an immunoassay strip, a (corresponding) timespan for a further fertility medical action indicating a temporal difference between the measures of estradiol and/or of luteinizing hormone and the (corresponding) further fertility medical action, and a (corresponding) status on whether or not the (corresponding) further fertility medical action resulted in (or contributed to) pregnancy of the (corresponding) further female and/or live birth. The status on whether or not the further fertility medical action resulted in pregnancy of the further female and/or live birth may comprise a vector of one or more numbers or characters attributed to various statuses. As an example, the status may comprise a binary value, wherein 0 relates to no pregnancy and 1 relates to pregnancy and live birth. Alternatively, or in addition, the status may comprise a score in terms of a number in the interval [0, 1] for success defined on a (continuous) scale. Alternatively, the timespan for the further fertility medical action can be replaced by a timestamp of the at least one further image of the further female's blood saliva sample and a timestamp of the further fertility medical action, as in this case the timespan for the further fertility medical action can be computed as a difference of the timestamps.

Each of the predetermined sets of data from the further females may further comprise items of meta data. Such items of meta data can advantageously be used to match the items of meta data of the female for whom the point in time of the fertility medical action shall be computed. Training the first pre-trained machine learning algorithm, the second pre-trained machine learning algorithm, and/or the third pre-trained machine learning algorithm may therefore also be based on the items of meta data from the further females. In so doing, a tailored and, hence, optimal point in time for the fertility medical action can be computed for the female.

As an example, the first pre-trained machine learning algorithm, the second pre-trained machine learning algorithm, and/or the third machine learning algorithm may have been trained on the server 160 once, every now and then (e.g. by a cronjob), or each time a new (predetermined) set of data from a further female is uploaded to the server 160 or when a batch of such (predetermined) sets of data has been uploaded to the server 160.

In an embodiment, the measure-to-point-in-time algorithm may be configured to compare the measure of estradiol in the blood or saliva sample and/or the measure of luteinizing hormone in the blood or saliva sample, and optionally, the one or more items of meta data to the predetermined sets of data from further females (e.g. from a database on a server 160). Alternatively, the measure-to-point-in-time algorithm may be configured to compare the at least one image of the blood or saliva sample, and optionally, the one or more items of meta data to the predetermined sets of data from further females. Each of the predetermined sets of data may comprise a measure of estradiol and/or a measure of luteinizing hormone (or alternatively, at least one further image of the blood or saliva sample) for a further female's blood or saliva sample processed on an immunoassay strip, a (corresponding) timespan for a (corresponding) further fertility medical action indicating a temporal difference between the measures of estradiol and/or of luteinizing hormone (e.g. inferred from a timestamp of the at least one image of the blood or saliva sample) and the (corresponding) further fertility medical action, and a (corresponding) status on whether or not the (corresponding) further fertility medical action resulted in (or contributed to) pregnancy of the (corresponding) further female and/or live birth. The status on whether or not the further fertility medical action resulted in pregnancy of the further female and/or live birth may comprise a vector of one or more numbers or characters attributed to various statuses. As an example, the status may comprise a binary value, wherein 0 relates to no pregnancy and 1 relates to pregnancy and live birth. Alternatively, or in addition, the status may comprise a score in terms of a number in the interval [0, 1] for success defined on a (continuous) scale. Alternatively, the timespan for the further fertility medical action can be replaced by a timestamp of the further fertility medical action and a timestamp of the measure of estradiol and/or the measure of luteinizing hormone (or alternatively, of the at least one further image of the blood or saliva sample) for a further female's blood or saliva sample processed on an immunoassay strip, as in this case the timespan for the further fertility medical action can be computed as a difference of the timestamps.

In this embodiment, the measure-to-point-in-time algorithm may further be configured to identify, in terms of a predetermined similarity criterion, one or more subsets of the predetermined sets of data from the further females that are similar to the measure of estradiol in the blood or saliva sample and/or to the measure of luteinizing hormone in the blood or saliva sample (or alternatively, to the at least one image of the blood or saliva sample) of the female, and optionally, to the one or more items of meta data, thereby gathering one or more timespans for the fertility medical action and corresponding one or more statuses. The predetermined similarity criterion may be based on a metric configured to measure distance in a (vector) space spanned by the data. The predetermined similarity criterion may comprise a threshold value or involve an optimization to minimize at least one distance in this (vector) space. The predetermined similarity criterion may be concatenated from several predetermined similarity subcriteria evaluating portions of the data.

In this embodiment, the measure-to-point-in-time algorithm may further be configured to compute an optimal timespan for the fertility medical action in the menstrual cycle of the female based on the one or more timespans for the fertility medical action and the corresponding one or more statuses. The measure-to-point-in-time algorithm may further be configured to compute 122, 124 the point in time for the fertility medical action in the menstrual cycle of the female by adding the optimal timespan for the fertility medical action to the timestamp of the at least one image of the blood or saliva sample. Computing the optimal timespan for the fertility medical action in the menstrual cycle of the female based on the one or more timespans for the fertility medical action and the corresponding one or more statuses may comprise clustering the one or more timespans for the fertility medical action into at least two classes (i.e. disjunct classes such as e.g. today, tomorrow, in two days, in three days, in four days, in five days or tomorrow in the morning, tomorrow in the afternoon etc.), wherein each class is/can be labelled by a corresponding timespan representing the one or more timespans for the fertility medical action in the class. Computing the optimal timespan for the fertility medical action in the menstrual cycle of the female based on the one or more timespans for the fertility medical action and the corresponding one or more statuses may further comprise computing for each class a probability of success (of the fertility medical action) based on the one or more statuses. As an example, one may sum over statuses per class or counting statuses that are deemed successful. Computing the optimal timespan for the fertility medical action in the menstrual cycle of the female based on the one or more timespans for the fertility medical action and the corresponding one or more statuses may further comprise identifying a class with the largest probability of success. Computing the optimal timespan for the fertility medical action in the menstrual cycle of the female based on the one or more timespans for the fertility medical action and the corresponding one or more statuses may further comprise defining the optimal timespan for the fertility medical action in the menstrual cycle of the female as the timespan corresponding to the class with the largest probability of success. Each of the predetermined sets of data from the further females may further comprise items of meta data. Again, such items of meta data can advantageously be used to match the items of meta data of the female for whom the point in time of the fertility medical action shall be computed. In so doing, a tailored and, hence, optimal point in time for the fertility medical action can be computed for the female.

The method 100 may further comprise computing 130 a point in time for an oocyte retrieval in the menstrual cycle of the female based in part on the point in time for the fertility medical action in the menstrual cycle of the female. Such may apply in particular in the case wherein the fertility medical action for which the point in time is computed is an oocyte maturation trigger stimulus. On the other hand, here and in the following, alternatively or in addition to computing 130 a point in time of the oocyte retrieval, the method may further comprise computing a point in time for a transfer of a fertilized egg into the female's uterus. Alternatively, or in addition, the method may further comprise computing a point in time for an embryo transfer. Alternatively, or in addition, the method may further comprise computing a point in time for an in-vivo insemination. Alternatively, or in addition, the method may further comprise computing a point in time for a timed sexual intercourse. In so doing, one may end up with a sequence of fertility medical actions. The point in time for an oocyte retrieval can be the point in time for the fertility medical action in the menstrual cycle of the female plus a predetermined timespan. As an example, in case of a human female, the oocyte maturation trigger stimulus can be a human chorionic gonadotropin (hCG) trigger stimulus/injection. Other oocyte maturation trigger stimuli for inducing oocyte maturation may e.g. be (or comprise) gonadotropin-releasing hormone agonist GnRHa, both GnRHa and hCG administered in combination, recombinant luteinizing hormone (LH), and/or kisspeptin. In other words, the oocyte maturation trigger stimulus can be (or comprise) human chorionic gonadotropin (hCG). Alternatively, or in addition, the oocyte maturation trigger stimulus can be (or comprise) gonadotropin-releasing hormone agonist GnRHa. Alternatively, or in addition, the oocyte maturation trigger stimulus can be (or comprise) a combination of GnRHa and hCG. Alternatively, or in addition, the oocyte maturation trigger stimulus can be (or comprise) recombinant luteinizing hormone (LH). Alternatively, or in addition, the oocyte maturation trigger stimulus can be (or comprise) kisspeptin. On the other hand, the substance to be injected as the oocyte maturation trigger may be equine chorionic gonadotropin (for horses), bovine chorionic gonadotropin (for cattle) etc. The predetermined timespan may be less than or equal to 30 hours, less than or equal to 32 hours, less than or equal to 34 hours, less than or equal to 36 hours, less than or equal to 38 hours, less than or equal to 40 hours, less than or equal to 42 hours, less than or equal to 44 hours. The point in time for the oocyte retrieval can be computed to increase the probability of pregnancy for the female and/or of live birth, optionally based on the predetermined sets of data from further females. In case of a human female, as of now, a predetermined timespan of 36 hours can be considered as an optimal predetermined timespan for human chorionic gonadotropin.

Alternatively, the point in time for the oocyte retrieval may be computed (as step 120) according to the method 100 with the fertility medical action being the oocyte retrieval, i.e. based on the at least one image of the blood or saliva sample and, optionally, the one or more items of meta data. In that case, the method 100 may further comprise computing (as step 130) a point in time of the oocyte maturation trigger stimulus. In any case, as already discussed, the method can be generalized to computing a sequence of one or more fertility medical actions.

The method 100 further comprises saving 140, under a unique identifier, the at least one image of the blood or saliva sample processed on the immunoassay strip 300 and the corresponding timestamp. In addition, the method 100 may further comprise saving 141, under a/the unique identifier, the one or more items of meta data. In addition, the method 100 may further comprise saving 142a, under a/the unique identifier, the measure of estradiol in the blood or saliva sample. In addition, the method 100 may further comprise saving 142b, under a/the unique identifier, the measure of estradiol in the blood or saliva sample and/or the measure of luteinizing hormone in the blood or saliva sample. In addition, the method 100 may further comprise saving 143, under a/the unique identifier, the point in time for the fertility medical action. In addition, the method 100 may further comprise obtaining 144a (e.g. via a user interface of the client computer 220 of the portable analysis kit 200) and saving 144b, under the unique identifier, an actual point in time for the fertility medical action (and/or a timespan between the actual point in time for the fertility medical action and e.g. the timestamp of the at least one image of the blood or saliva sample). In addition, the method 100 may further comprise saving 145, under a/the unique identifier, the point in time for the oocyte retrieval (or any other fertility medical action) and, optionally, obtaining 146a (e.g. via a user interface of the client computer 220 of the portable analysis kit 200) and saving 146b, under the unique identifier, an actual point in time for the oocyte retrieval (and/or a timespan between the actual point in time for the oocyte retrieval and e.g. the timestamp of the at least one image of the blood or saliva sample). In addition, the method 100 may further comprise obtaining 147a (e.g. via a user interface of the client computer 220 of the portable analysis kit 200) and saving 147b, under a/the unique identifier, a status of pregnancy of the female.

In addition, the method 100 may further comprise obtaining 148a (approximately more than 40 weeks after the fertility medical action and e.g. via a user interface of the client computer 220 of the portable analysis kit 200 or a browser interface) and saving 148b, under a/the unique identifier, a status of live birth. Such a status or such statuses can be essential in order for the method/the algorithms to learn to compute the (optimal) point in time of the fertility medical action. In fact, the method 100 may comprise prompting (e.g. via a user interface of the client computer 220 or via a browser interface) the female (or another user) to input the actual point in time for the fertility medical action, the actual point in time for the oocyte retrieval, the actual point in time for the oocyte maturation trigger stimulus, the status of pregnancy of the female, and/or the status of live birth. It is also conceivable to extend the method 100 so as to also analyze another immunoassay strip (e.g. based on hCG detection), thereby automatically obtaining the status of pregnancy of the female. As an example, an additional testing zone e.g. for hCG detection may be provided on the immunoassay strip 300. As another example, the additional testing zone e.g. for hCG detection may be provided on another immunoassay strip e.g. configured to be analyzed in the stage (231) of the portable analysis device 230.

Saving the aforementioned data may comprise transmitting the data to the server 160 and saving data on the server 160. Such data can advantageously be used for shallow/deep learning, thereby improving the precision of the point in time of the fertility medical action.

The method 100 further comprises retraining 150 the first pre-trained machine learning algorithm (in part) based on the aforementioned saved data. In addition, the method 100 may further comprise retraining 151 the second pre-trained machine learning algorithm (in part) based on the aforementioned saved data. In addition, the method 100 may further comprise retraining 152 the third pre-trained machine learning algorithm (in part) based on the aforementioned saved data. In addition, the method 100 may further comprise adding 153 to the predetermined sets of data from further females, as a new set of data, the aforementioned saved data. As an example, the first pre-trained machine learning algorithm, the second pre-trained machine learning algorithm, and/or the third machine learning algorithm may be trained e.g. on the server 160 once, every now and then (e.g. by a cronjob), or each time a new (predetermined) set of data from a further female is uploaded to the server 160 or when a batch of such (predetermined) sets of data has been uploaded to the server 160, wherein the aforementioned saved data of the female is promoted to a (new predetermined) set of data to be added to the predetermined sets of data from the further females. Put differently, the female has now become one of the further females and the method 100 can be repeated in order to compute a point in time for a fertility medical action in a menstrual cycle of another female. Such a closed-loop process is schematically illustrated by respective arrows pointing back to step 110 in **Fig. 1a****-c.**

There is provided a server 160 in a network, wherein the server 160 is configured to run the method 100 for computing a point in time for a fertility medical action in a menstrual cycle of a female of one of the preceding embodiments. As an example, the network may be the internet. The at least one image of the blood or saliva sample processed on the immunoassay strip 300 and, optionally, the one or more items of meta data, and optionally, the status of pregnancy of the female, and optionally, the status of live birth may be obtained via a client computer 220 of a portable analysis kit 200. The server 160 may provide the point in time for the fertility medical action in the menstrual cycle of the female to the client computer 220 of the portable analysis kit 200. The server 160 may further be configured for learning (e.g. re-training the analysis algorithm, ...). In order to do so, and if necessary (e.g. in case of deep learning), the server 160 may comprise dedicated hardware for the learning process. Alternatively, the learning process can be outsourced and carried out elsewhere (e.g. on a machine with dedicated hardware). In this case, only the re-trained algorithm (e.g. the analysis algorithm) may be uploaded to and installed on the server 160.

Alternatively, the computer-implemented method 100 for computing the point in time for the fertility medical action in the menstrual cycle of the female may be run on a/the client computer 220 (i.e. not on a server). In that case, it may be advantageous to download the one or more aforementioned algorithms (e.g. the analysis algorithm, the pre-trained machine learning algorithms etc.) to the client computer 220. In so doing, the point in time for the fertility medical action can be computed offline. Still, the computed point in time for the fertility medical action and input data may be uploaded to the server. This may be advantageous in order to successively improve the algorithms.

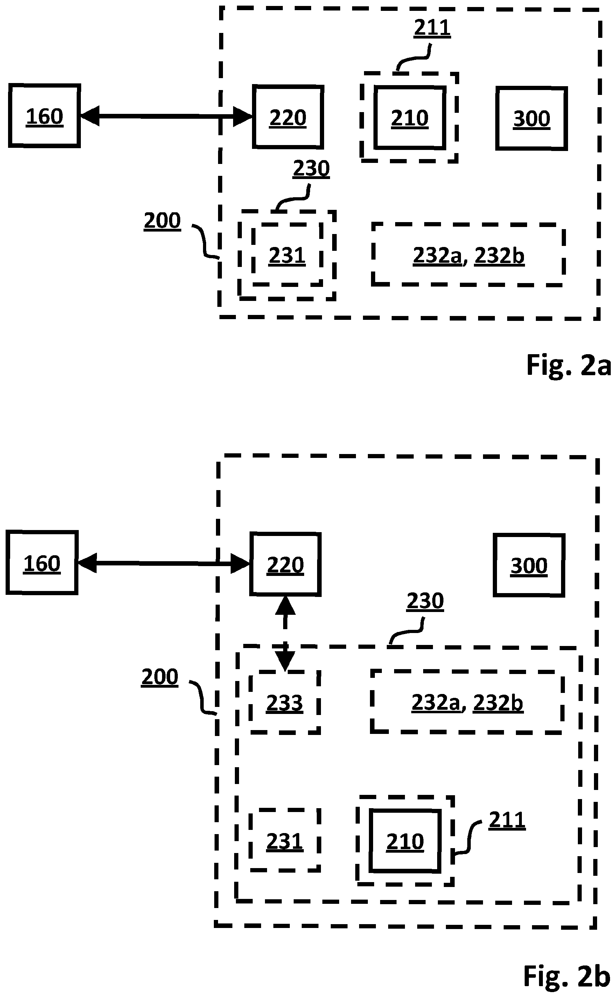

There is provided a portable analysis kit 200, as schematically illustrated in **Fig. 2a****,** comprising an immunoassay strip 300 configured to process a blood or saliva sample, at least one image sensor 210 configured to take, and optionally timestamp (i.e. thereby producing a timestamp), at least one image of the blood or saliva sample processed on the immunoassay strip 300, and a client computer 220 configured to output (e.g. on a user interface, in particular on a graphical output/display) a point in time for a fertility medical action in a menstrual cycle of a female computed based on the at least one image of the blood or saliva sample processed on the immunoassay strip 300. The point in time for the fertility medical action in the menstrual cycle of the female may be computed according to the computer-implemented method 100. The computation does not have to be done on the client computer 220 as it can be done on the server 160. In fact, the client computer 220 may further be configured to communicate with the server 160 in the network, wherein the point in time for the fertility medical action in the menstrual cycle of the female may be computed on the server 160. "Client" in "client computer" refers to a client-server pair. "Client" shall not be construed as necessarily referring to a patient. In fact, as examples, the client computer 220 may be used by a (human) female (i.e. a patient) and/or by another person (e.g. her doctor, partner). The client computer 220 may comprise a user interface for input (e.g. the one or more items of meta data) and/or output (e.g. the point in time for the fertility medical action). The immunoassay strip 300 may e.g. be a lateral flow immunochromatographic assay for quantitatively testing a blood or saliva sample for one, two or more hormones. The client computer 220 may be a smart device, optionally a smartphone or a tablet. Alternatively, the client computer 220 may be e.g. a desktop computer or a workstation.

The client computer 220 of the portable analysis kit 200 may comprise a user interface configured to enable a user (e.g. a female patient and/or a doctor) of the client computer 220 to enter input data (e.g. via a keyboard or a touchscreen) and to receive output data (e.g. via a display or the touchscreen). The server 160 may also provide an interface configured to receive the input data from the user interface of the client computer 220 of the portable analysis kit 200 and to send the output data to the user interface of the client computer 220 of the portable analysis kit 200. The input data may comprise the at least one image of a blood or saliva sample processed on an immunoassay strip 300. The input data may comprise the one or more items of meta data. The input data may comprise the actual point in time for the fertility medical action. The input data may comprise the actual point in time for the oocyte retrieval (if any). The input data may comprise the status of pregnancy of the female. The input data may comprise the status of live birth. The input data may comprise information specifying the one or more additional fertility medical actions or the next fertility medical action in the sequence of fertility medical actions. As an example, such information may specify that the next fertility medical action shall be an oocyte retrieval (e.g. after an oocyte maturation trigger stimulus). The output data may comprise the point in time for the fertility medical action in the menstrual cycle of the female (and e.g. a corresponding probability of confidence). The output data may comprise the measure of estradiol in the blood or saliva sample. The output data may comprise the measure of estradiol in the blood or saliva sample and/or the measure of luteinizing hormone in the blood or saliva sample. The output data may comprise the point in time for the oocyte retrieval in the menstrual cycle of the female (and e.g. a corresponding probability of confidence). The output data may comprise instruction data such as e.g. to repeat the test at a given time. In general, such interfaces may prove useful for customization of various kinds (e.g. timezone settings).

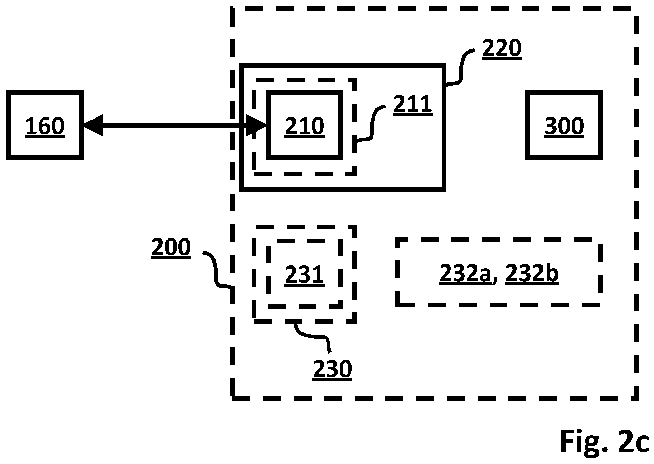

As schematically illustrated in **Fig. 2c****,** the smart device may comprise the at least one image sensor 210. In other words, the smart device, e.g. the smartphone, can be used to take the at least one image of the blood or saliva sample processed on the immunoassay strip 300. The smart device, e.g. the smartphone, may be combined with at least one magnificent lens 232b configured to magnify the blood or saliva sample processed on the immunoassay strip 300 (optionally held on the stage 231) before taking the at least one image of the blood or saliva sample processed on the immunoassay strip 300.

Alternatively, or in addition, the portable analysis kit 200 may further comprise a portable analysis device 230, as schematically illustrated e.g. in **Fig. 2b****,** wherein the portable analysis device 230 may comprise a stage 231 to hold the immunoassay strip 300 configured to process the blood or saliva sample. The portable analysis device 230 may (or may not) further comprise the at least one image sensor 210. The portable analysis device 230 may further comprise a microscope 232a or at least one magnificent lens 232b configured to magnify the blood or saliva sample processed on the immunoassay strip 300 held on the stage 231 before taking the at least one image of the blood or saliva sample processed on the immunoassay strip 300.

Due to the microscope 232a or to the at least one magnificent lens 232b the precision of analyzing the blood or saliva sample processed on the immunoassay strip 300 and, eventually, the precision of the point in time of the fertility, can be increased.

The portable analysis device 230 may further comprise at least one communication interface 233 configured to connect (e.g. via a USB) to the client computer 220, optionally to the smart device. A wired connection may advantageously be used to power the portable analysis device 230. Alternatively, or in addition, the at least one communication interface 233 may be configured to connect wirelessly (e.g. via Wi-Fi, Bluetooth, NFC, ...) to the client computer 220, optionally to the smart device. In any case, the wired or wireless connection allows for communication according to a given protocol. As an example, this connection may be used to transmit the at least one image of the blood or saliva sample processed on the immunoassay strip 300 from the portable analysis device 230 to the client computer 220.

The portable analysis kit 200 may further comprise a digital camera 211, wherein the digital camera 211 may comprise the at least one image sensor 210, and optionally wherein the digital camera 211 may comprise an optical zoom and/or a digital zoom. Alternatively, as in **Fig. 2b****,** (or in addition) the portable analysis device 230 may comprise a digital camera 211, wherein the digital camera 211 may comprise the at least one image sensor 210, and optionally wherein the digital camera 211 may comprise an optical zoom and/or a digital zoom. The at least one image sensor 210 may comprise a CCD sensor. Alternatively, or in addition the at least one image sensor 210 may comprise a CMOS sensor. Alternatively, or in addition the at least one image sensor 210 may comprise a magnetic image sensor.

The immunoassay strip 300 may be a lateral flow immunochromatographic assay. As shown in **Fig. 3a****-c,** the immunoassay strip 300 may comprise at least one first tine 320, 320a (e.g. aligned along the lateral flow immunoassay 300), wherein the at least one first tine 320, 320a may comprise one or more testing zones. As shown in **Fig. 3c****,** the immunoassay strip 300 may comprise at least one second tine 320b (e.g. parallel to the at least one first tine 320, 320a), wherein the at least one second tine 320b may comprise one or more testing zones.

As in **Fig. 3a****-c,** at least one first testing zone 303 out of the one or more testing zones of the at least one first tine 320, 320a may be configured to react with a first hormone of the blood or saliva sample processed on the immunoassay strip 300. Alternatively, or in addition, as in **Fig. 3b****,** at least one second testing zone 304 out of the one or more testing zones of the at least one first tine 320, 320a may be configured to react with a second hormone of the blood or saliva sample processed on the immunoassay strip 300. Alternatively, or in addition, as in **Fig. 3c****,** at least one second testing zone 304 out of the one or more testing zones of the at least one second tine 320b may be configured to react with a second hormone of the blood or saliva sample processed on the immunoassay strip 300. Alternatively, or in addition, one or more of the testing zones could also react with a first hormone and/or second hormone. Testing two hormones on the immunoassay strip 300 is advantageous as the precision of the analysis and, hence, the point in time for the fertility medical action can be increased.

The at least one first testing zone 303 may undergo a change in color and/or intensity as a function of a concentration of the first hormone deposited in the at least one first testing zone 303 as the blood or saliva sample is processed on the immunoassay strip 300. Alternatively, or in addition, the at least one second testing zone 304 may undergo a change in color and/or intensity as a function of a concentration of the first hormone deposited in the at least one second testing zone 304 as the blood or saliva sample is processed on the immunoassay strip 300. A change in color and/or intensity may be homogeneous or heterogeneous per (first/second) testing zone. A change in color and/or intensity and a corresponding pattern resulting from the first testing zones 303 and the second testing zones 304 may be analyzed via image recognition (e.g. in the analysis algorithm) in order to correlate it with one or more hormone levels in the blood or saliva sample processed on the immunoassay strip 300.

The first hormone of the blood or saliva sample may be estradiol (or another estrogen). The second hormone of the blood or saliva sample may be luteinizing hormone. As shown in **Fig. 4****,** levels of estradiol (E2) and luteinizing hormone (LH) plotted against time in a menstrual cycle of a female may peak at different points in time. An immunoassay strip configured to test both estradiol (E2) and luteinizing hormone (LH) can therefore increase the precision for, eventually, computing a point in time for the fertility medical action.

The at least one first tine 320, 320a may comprise at least one reference zone. The at least one second tine 320b may comprise at least one reference zone. Reference zones (not shown in **Fig. 3a****-c**) may advantageously be used e.g. for calibration of the colormap and/or the grayscale to account e.g. for varying lightning conditions. Furthermore, the immunoassay strip 300 may comprise at least one fiducial 308 which can be of use in image-correction. Furthermore, the immunoassay strip 300 may comprise at least one additional testing zone for testing a status of pregnancy. As an example, and in case of a human female, the at least one additional testing zone may be configured to test human chorionic gonadotropin (hCG).

### REFERENCE NUMERALS

- 100: computer-implemented method for computing a point in time for a fertility medical action in a menstrual cycle of a female
- 110: obtaining at least one image of a blood or saliva sample processed on an immunoassay strip
- 111: obtaining one or more items of meta data
- 120: computing the point in time for the fertility medical action in the menstrual cycle of the female in part based on the at least one image of the blood or saliva sample processed on the immunoassay strip
- 121: determining a measure of estradiol in the blood or saliva sample based on the at least one image of the blood or saliva sample
- 122: computing the point in time for the fertility medical action in the menstrual cycle of the female in part based on the measure of estradiol in the blood or saliva sample
- 123: determining a measure of estradiol in the blood or saliva sample and a measure of luteinizing hormone in the blood or saliva sample based on the at least one image of the blood or saliva sample
- 124: computing the point in time for the fertility medical action in the menstrual cycle of the female in part based on the measure of estradiol in the blood or saliva sample and on the measure of luteinizing hormone in the blood or saliva sample
- 130: computing a point in time for an oocyte retrieval in the menstrual cycle of the female based in part on the point in time for the fertility medical action in the menstrual cycle of the female
- 140: saving the at least one image of the blood or saliva sample processed on the immunoassay strip and the corresponding timestamp
- 141: saving the one or more items of meta data
- 142a: saving the measure of estradiol in the blood or saliva sample
- 142b: saving the measure of estradiol in the blood or saliva sample and/or the measure of luteinizing hormone in the blood or saliva sample
- 143: saving the point in time for the fertility medical action
- 144a: obtaining an actual point in time for the fertility medical action
- 144b: saving an actual point in time for the fertility medical action
- 145: saving the point in time for the oocyte retrieval
- 146a: obtaining an actual point in time for the oocyte retrieval
- 146b: saving an actual point in time for the oocyte retrieval
- 147a: obtaining a status of pregnancy of the female
- 147b: saving a status of pregnancy of the female
- 148a: obtaining a status of live birth
- 148b: saving a status of live birth
- 150: retraining the first pre-trained machine learning algorithm
- 151: retraining the second pre-trained machine learning algorithm
- 152: retraining the third pre-trained machine learning algorithm
- 153: adding data to the predetermined sets of data from further females
- 160: server
- 200: portable analysis kit
- 210: image sensor
- 211: digital camera
- 220: client computer
- 230: portable analysis device
- 231: stage
- 232a: microscope
- 232b: magnificent lens
- 233: communication interface
- 300: immunoassay strip
- 303: first testing zone
- 304: second testing zone
- 308: fiducial
- 320, 320a: first tine
- 320b: second tine

## Claims

1. A computer-implemented method (100) for computing a point in time for a fertility medical action in a menstrual cycle of a female, comprising:
- obtaining (110) at least one image of a blood or saliva sample processed on an immunoassay strip (300); and
- computing (120) the point in time for the fertility medical action in the menstrual cycle of the female in part based on the at least one image of the blood or saliva sample processed on the immunoassay strip (300), comprising:
- applying the at least one image of the blood or saliva sample to an analysis algorithm, wherein the analysis algorithm comprises a first pre-trained machine learning algorithm configured to compute the point in time for the fertility medical action in the menstrual cycle of the female;
the method (100) further comprising
- saving data, comprising:
- saving (140), under a unique identifier, the at least one image of the blood or saliva sample processed on the immunoassay strip (300) and the corresponding timestamp;
the method (100) further comprising:
- retraining (150) the first pre-trained machine learning algorithm in part based on the saved data.

2. The method (100) of claim 1, wherein the point in time for the fertility medical action is computed to increase the probability of pregnancy for the female and/or of live birth after the fertility medical action.

3. The method (100) of claim 1 or 2, wherein the fertility medical action is an oocyte maturation trigger stimulus, optionally wherein the oocyte maturation trigger stimulus induces final oocyte maturation.

4. The method (100) of one of the preceding claims, wherein obtaining (110) the at least one image of the blood or saliva sample processed on the immunoassay strip (300) comprises obtaining the at least one image of the blood or saliva sample processed on the immunoassay strip (300) via at least one image sensor (210) of a portable analysis kit (200), optionally wherein the at least one image of the blood or saliva sample processed on the immunoassay strip (300) has a timestamp.

5. The method (100) of one of the preceding claims, wherein computing (120) the point in time for the fertility medical action in the menstrual cycle of the female in part based on the at least one image of the blood or saliva sample processed on the immunoassay strip (300) comprises:
- applying the one or more items of meta data to the analysis algorithm.

6. The method (100) of one of the preceding claims, wherein the first pre-trained machine learning algorithm comprises an artificial neural network and/or a convolutional neural network.

7. The method (100) of one of the preceding claims, wherein computing (120) the point in time for the fertility medical action in the menstrual cycle of the female in part based on the at least one image of the blood or saliva sample processed on the immunoassay strip (300) comprises:
- determining (121) a measure of estradiol in the blood or saliva sample based on the at least one image of the blood or saliva sample;
- computing (122) the point in time for the fertility medical action in the menstrual cycle of the female in part based on the measure of estradiol in the blood or saliva sample.

8. The method (100) of one of the claims 1 to 6, wherein computing (120) the point in time for the fertility medical action in the menstrual cycle of the female in part based on the at least one image of the blood or saliva sample processed on the immunoassay strip (300) comprises:
- determining (123) a measure of estradiol in the blood or saliva sample and a measure of luteinizing hormone in the blood or saliva sample based on the at least one image of the blood or saliva sample;
- computing (124) the point in time for the fertility medical action in the menstrual cycle of the female in part based on the measure of estradiol in the blood or saliva sample and on the measure of luteinizing hormone in the blood or saliva sample.

9. The method (100) of one of the preceding claims, when dependent on claim 6, wherein the first pre-trained machine learning algorithm has been trained in part based on predetermined sets of data from further females, optionally wherein each of the predetermined sets of data comprises at least one further image of a further female's blood or saliva sample processed on an immunoassay strip, a timespan for a further fertility medical action indicating a temporal difference between the at least one further image and the further fertility medical action, and a status on whether or not the further fertility medical action resulted in pregnancy of the further female and/or live birth.

10. The method (100) of one of the preceding claims, wherein saving the data further comprises:
- saving (143), under the unique identifier, the point in time for the fertility medical action; and
- optionally, saving (141), under the unique identifier, the one or more items of meta data; and
- optionally, obtaining (144a) and saving (144b), under the unique identifier, an actual point in time for the fertility medical action; and
- optionally, obtaining (147a) and saving (147b), under the unique identifier, a status of pregnancy of the female; and
- optionally, obtaining (148a) and saving (148b), under the unique identifier, a status of live birth.

11. The method (100) of one of the preceding claims, when dependent on claim 9, further comprising:
- adding (153) to the predetermined sets of data from further females, as a new set of data, the saved data.

12. The method (100) of one of the preceding claims, wherein the computed (120) point in time for the fertility medical action is:
- a timestamp specifying a day of the calendar and/or an hour of the day and/or a minute of the hour in an appropriate timezone; or
- a day of the calendar, optionally specifying time intervals such as morning, afternoon, evening, and/or night.

13. A server (160) in a network, wherein the server (160) is configured to run the method (100) for computing a point in time for a fertility medical action in a menstrual cycle of a female of one of the preceding claims, optionally wherein:
- the at least one image of the blood or saliva sample processed on the immunoassay strip (300); and
- optionally, the one or more items of meta data; and
- optionally, the status of pregnancy of the female; and
- optionally, the status of live birth
are obtained via a client computer (220) of a portable analysis kit (200), and wherein the server (160) provides the point in time for the fertility medical action in the menstrual cycle of the female to the client computer (220) of the portable analysis kit (200).

14. A portable analysis kit (200), comprising:
- an immunoassay strip (300) configured to process a blood or saliva sample; and
- at least one image sensor (210) configured to take, and optionally timestamp, at least one image of the blood or saliva sample processed on the immunoassay strip (300); and
- a client computer (220) configured to output a point in time for a fertility medical action in a menstrual cycle of a female computed based on the at least one image of the blood or saliva sample processed on the immunoassay strip (300);
wherein the point in time for the fertility medical action in the menstrual cycle of the female is computed according to the method of one of the claims 1 to 12, optionally wherein the client computer (220) is further configured to communicate with a server (160) in a network according to claim 13, and optionally wherein the point in time for the fertility medical action in the menstrual cycle of the female is computed on the server (160) according to the method of one of the claims 1 to 12.

15. The portable analysis kit (200) of claim 14, further comprising a portable analysis device (230), wherein the portable analysis device (230) comprises a stage (231) to hold the immunoassay strip (300) configured to process the blood or saliva sample, optionally wherein the portable analysis device (230) further comprises the at least one image sensor (210).

## Patentansprüche

1. Computerimplementiertes Verfahren (100) zum Berechnen eines Zeitpunkts für eine medizinische Fertilitätsmaßnahme in einem Menstruationszyklus eines Weibchen, umfassend:
- Erhalten (110) mindestens eines Bildes einer auf einem Immunoassay-Streifen (300) verarbeiteten Blut- oder Speichelprobe; und
- Berechnen (120) des Zeitpunkts für die medizinische Fertilitätsmaßnahme im Menstruationszyklus des Weibchens teilweise basierend auf dem mindestens einen Bild der auf dem Immunoassay-Streifen (300) verarbeiteten Blut- oder Speichelprobe, umfassend:
- Anwenden des mindestens einen Bildes der Blut- oder Speichelprobe zu einem Analysealgorithmus, wobei der Analysealgorithmus einen ersten vortrainierten Maschinenlernalgorithmus umfasst, der dazu ausgelegt ist, den Zeitpunkt für die medizinische Fertilitätsmaßnahme im Menstruationszyklus des Weibchens zu berechnen;
wobei Verfahren (100) ferner Folgendes umfasst:
- Speichern von Daten, umfassend:
- Speichern (140) des mindestens einen Bildes der auf dem Immunoassay-Streifen (300) verarbeiteten Blut- oder Speichelprobe und des entsprechenden Zeitstempels unter einer eindeutigen Kennung;
wobei das Verfahren (100) ferner Folgendes umfasst:
- erneutes Trainieren (150) des ersten vortrainierten Maschinenlernalgorithmus teilweise basierend auf den gespeicherten Daten.

2. Verfahren (100) nach Anspruch 1, wobei der Zeitpunkt für die medizinische Fertilitätsmaßnahme berechnet wird, um die Wahrscheinlichkeit einer Schwangerschaft für das Weibchen und/oder einer Lebendgeburt nach der medizinischen Fertilitätsmaßnahme zu erhöhen.

3. Verfahren (100) nach Anspruch 1 oder 2, wobei die medizinische Fertilitätsmaßnahme ein Eizellreifungsauslösestimulus ist, wobei optional der Eizellreifungsauslösestimulus eine endgültige Eizellreifung induziert.

4. Verfahren (100) nach einem der vorhergehenden Ansprüche, wobei das Erhalten (110) des mindestens einen Bildes der auf dem Immunoassay-Streifen (300) verarbeiteten Blut- oder Speichelprobe Erhalten des mindestens einen Bildes der auf dem Immunoassay-Streifen (300) verarbeiteten Blut- oder Speichelprobe über mindestens einen Bildsensor (210) eines tragbaren Analysekits (200) umfasst, wobei optional das mindestens eine Bild der auf dem Immunoassay-Streifen (300) verarbeiteten Blut- oder Speichelprobe einen Zeitstempel aufweist.

5. Verfahren (100) nach einem der vorhergehenden Ansprüche, wobei das Berechnen (120) des Zeitpunkts für die medizinische Fertilitätsmaßnahme im Menstruationszyklus des Weibchens teilweise basierend auf dem mindestens einen Bild der auf dem Immunoassay-Streifen (300) verarbeiteten Blut- oder Speichelprobe Folgendes umfasst:
- Anwenden des einen oder der mehreren Metadatenelemente zum Analysealgorithmus.

6. Verfahren (100) nach einem der vorhergehenden Ansprüche, wobei der erste vortrainierte Maschinenlernalgorithmus ein künstliches neuronales Netzwerk und/oder ein Convolutional Neuronal Network umfasst.

7. Verfahren (100) nach einem der vorhergehenden Ansprüche, wobei das Berechnen (120) des Zeitpunkts für die medizinische Fertilitätsmaßnahme im Menstruationszyklus des Weibchens teilweise basierend auf dem mindestens einen Bild der auf dem Immunoassay-Streifen (300) verarbeiteten Blut- oder Speichelprobe Folgendes umfasst:
- Bestimmen (121) eines Messwerts für Östradiol in der Blut- oder Speichelprobe basierend auf dem mindestens einen Bild der Blut- oder Speichelprobe;
- Berechnen (122) des Zeitpunkts für die medizinische Fertilitätsmaßnahme im Menstruationszyklus des Weibchens teilweise basierend auf dem Messwert für Östradiol in der Blut- oder Speichelprobe.

8. Verfahren (100) nach einem der Ansprüche 1 bis 6, wobei das Berechnen (120) des Zeitpunkts für die medizinische Fertilitätsmaßnahme im Menstruationszyklus des Weibchens teilweise basierend auf dem mindestens einen Bild der auf dem Immunoassay-Streifen (300) verarbeiteten Blut- oder Speichelprobe Folgendes umfasst:
- Bestimmen (123) eines Messwerts für Östradiol in der Blut- oder Speichelprobe und eines Messwerts für luteinisierendes Hormon in der Blut- oder Speichelprobe basierend auf dem mindestens einen Bild der Blut- oder Speichelprobe;
- Berechnen (124) des Zeitpunkts für die medizinische Fertilitätsmaßnahme im Menstruationszyklus des Weibchens teilweise basierend auf dem Messwert für Östradiol in der Blut- oder Speichelprobe und dem Messwert für luteinisierendes Hormon in der Blut- oder Speichelprobe.

9. Verfahren (100) nach einem der vorhergehenden Ansprüche, wenn abhängig von Anspruch 6, wobei der erste vortrainierte Maschinenlernalgorithmus teilweise basierend auf vorbestimmten Datensätzen von weiteren Weibchen trainiert wurde, wobei optional jeder der vorbestimmten Datensätze mindestens ein weiteres Bild einer auf einem Immunoassay-Streifen verarbeiteten Blut- oder Speichelprobe eines weiteren Weibchens, eine Zeitspanne für eine weitere medizinische Fertilitätsmaßnahme, die einen zeitlichen Unterschied zwischen dem mindestens einen weiteren Bild und der weiteren medizinischen Fertilitätsmaßnahme angibt, und einen Status dessen umfasst, ob die weitere medizinische Fertilitätsmaßnahme zu einer Schwangerschaft des weiteren Weibchens und/oder einer Lebendgeburt führte oder nicht.

10. Verfahren (100) nach einem der vorhergehenden Ansprüche, wobei das Speichern der Daten ferner Folgendes umfasst:
- Speichern (143) des Zeitpunkts für die medizinische Fertilitätsmaßnahme unter der eindeutigen Kennung; und
- optional Speichern (141) des einen oder der mehreren Metadatenelemente unter der eindeutigen Kennung; und
- optional Erhalten (144a) und Speichern (144b) eines tatsächlichen Zeitpunkts für die medizinische Fertilitätsmaßnahme unter der eindeutigen Kennung; und
- optional Erhalten (147a) und Speichern (147b) eines Schwangerschaftsstatus des Weibchens unter der eindeutigen Kennung; und
- optional Erhalten (148a) und Speichern (148b) eines Status einer Lebendgeburt unter der eindeutigen Kennung.

11. Verfahren (100) nach einem der vorhergehenden Ansprüche, wenn abhängig von Anspruch 9, ferner umfassend:
- Hinzufügen (153) der gespeicherten Daten zu den vorbestimmten Datensätzen von weiteren Weibchen als neuen Datensatz.

12. Verfahren (100) nach einem der vorhergehenden Ansprüche, wobei es sich bei dem berechneten (120) Zeitpunkt für die medizinische Fertilitätsmaßnahme um Folgendes handelt:
- einen Zeitstempel, der einen Tag des Kalenders und/oder eine Stunde des Tages und/oder eine Minute der Stunde in einer geeigneten Zeitzone angibt; oder
- einen Tag des Kalenders, optional unter Angabe von Zeitintervallen wie morgens, nachmittags, abends und/oder nachts.

13. Server (160) in einem Netzwerk, wobei der Server (160) dazu ausgelegt ist, das Verfahren (100) zum Berechnen eines Zeitpunkts für eine medizinische Fertilitätsmaßnahme in einem Menstruationszyklus eines Weibchens nach einem der vorhergehenden Ansprüche auszuführen, wobei optional:
- das mindestens eine Bild der auf dem Immunoassay-Streifen (300) verarbeiteten Blut- oder Speichelprobe; und
- optional das eine oder die mehreren Metadatenelemente; und
- optional der Schwangerschaftsstatus des Weibchens und
- optional der Status der Lebendgeburt
über einen Client-Computer (220) eines tragbaren Analysekits (200) erhalten werden, und wobei der Server (160) dem Client-Computer (220) des tragbaren Analysekits (200) den Zeitpunkt für die medizinische Fertilitätsmaßnahme im Menstruationszyklus des Weibchens bereitstellt.

14. Tragbares Analysekit (200), umfassend:
- einen Immunoassay-Streifen (300), der dazu ausgelegt ist, eine Blut- oder Speichelprobe zu verarbeiten; und
- mindestens einen Bildsensor (210), der dazu ausgelegt ist, mindestens ein Bild der auf dem Immunoassaystreifen (300) verarbeiteten Blut- oder Speichelprobe aufzunehmen und optional mit einen Zeitstempel zu versehen; und
- einen Client-Computer (220), der dazu ausgelegt ist, einen Zeitpunkt für eine medizinische Fertilitätsmaßnahme in einem Menstruationszyklus eines Weibchens auszugeben, der basierend auf dem mindestens einen Bild der auf dem Immunoassay-Streifen (300) verarbeiteten Blut- oder Speichelprobe berechnet wird;
wobei der Zeitpunkt für die medizinische Fertilitätsmaßnahme im Menstruationszyklus des Weibchens gemäß dem Verfahren nach einem der Ansprüche 1 bis 12 berechnet wird, wobei optional der Client-Computer (220) ferner dazu ausgelegt ist, mit einem Server (160) in einem Netzwerk nach Anspruch 13 zu kommunizieren, und wobei optional der Zeitpunkt für die medizinische Fertilitätsmaßnahme im Menstruationszyklus des Weibchens auf dem Server (160) gemäß dem Verfahren nach einem der Ansprüche 1 bis 12 berechnet wird.

15. Tragbares Analysekit (200) nach Anspruch 14, ferner umfassend eine tragbare Analysevorrichtung (230), wobei die tragbare Analysevorrichtung (230) eine Plattform (231) zum Halten des Immunoassay-Streifens (300) umfasst, der zum Verarbeiten der Blut- oder Speichelprobe ausgelegt ist, wobei die tragbare Analysevorrichtung (230) optional ferner den mindestens einen Bildsensor (210) umfasst.

## Revendications

1. Procédé effectué par ordinateur (100) pour calculer un instant t dans le temps pour une procédure médicale de fertilité dans un cycle menstruel d'une femelle, comprenant :
- l'obtention (110) d'au moins une image d'un échantillon de sang ou de salive traité sur une bandelette de test immunologique (300) ; et
- le calcul (120) du point dans le temps pour la procédure médicale de fertilité dans le cycle menstruel de la femelle en partie sur la base de l'au moins une image de l'échantillon de sang ou de salive traité sur la bandelette de test immunologique (300), comprenant :
- l'application de l'au moins une image de l'échantillon de sang ou de salive à un algorithme d'analyse, l'algorithme d'analyse comprenant un premier algorithme d'apprentissage automatique pré-entraîné configuré pour calculer l' instant t dans le temps de la procédure médicale de fertilité dans le cycle menstruel de la femelle ;
le procédé (100) comprenant en outre
- l'enregistrement de données, comprenant :
- l'enregistrement (140), sous un identifiant unique, de l'au moins une image de l'échantillon de sang ou de salive traité sur la bandelette de test immunologique (300) et de l'horodatage correspondant ;
le procédé (100) comprenant en outre :
- le réentraînement (150) du premier algorithme d'apprentissage automatique pré-entraîné en partie sur la base des données sauvegardées.

2. Procédé (100) selon la revendication 1, l' instant t dans le temps pour la procédure médicale de fertilité étant calculé pour augmenter la probabilité de grossesse pour la femelle et/ou de naissance vivante après la procédure médicale de fertilité.

3. Procédé (100) selon la revendication 1 ou 2, la procédure médicale de fertilité étant un stimulus déclencheur de la maturation des ovocytes, éventuellement le stimulus déclencheur de la maturation des ovocytes induisant la maturation finale des ovocytes.

4. Procédé (100) selon l'une des revendications précédentes, l'obtention (110) de l'au moins une image de l'échantillon de sang ou de salive traité sur la bandelette de test immunologique (300) comprenant l'obtention de l'au moins une image de l'échantillon de sang ou de salive traité sur la bandelette de test immunologique (300) par l'intermédiaire d'au moins un capteur d'image (210) d'un kit d'analyse portable (200), éventuellement, l'au moins une image de l'échantillon de sang ou de salive traité sur la bandelette de test immunologique (300) comportant un horodatage.

5. Procédé (100) selon l'une des revendications précédentes, le calcul (120) de l' instant t dans le temps pour la procédure médicale de fertilité dans le cycle menstruel de la femelle, en partie sur la base de l'au moins une image de l'échantillon de sang ou de salive traité sur la bandelette de test immunologique (300), comprenant :
- l'application du ou des éléments de métadonnées à l'algorithme d'analyse.

6. Procédé (100) selon l'une des revendications précédentes, le premier algorithme d'apprentissage automatique pré-entraîné comprenant un réseau neuronal artificiel et/ou un réseau neuronal convolutif.

7. Procédé (100) selon l'une des revendications précédentes, le calcul (120) de l' instant t dans le temps pour la procédure médicale de fertilité dans le cycle menstruel de la femelle, en partie sur la base de l'au moins une image de l'échantillon de sang ou de salive traité sur la bandelette de test immunologique (300), comprenant :
- la détermination (121) d'une mesure de l'estradiol dans l'échantillon de sang ou de salive sur la base de l'au moins une image de l'échantillon de sang ou de salive ;
- le calcul (122) de l' instant t dans le temps pour la procédure médicale de fertilité dans le cycle menstruel de la femelle en partie sur la base de la mesure de l'estradiol dans l'échantillon de sang ou de salive.

8. Procédé (100) selon l'une des revendications 1 à 6, le calcul (120) de l' instant t dans le temps pour la procédure médicale de fertilité dans le cycle menstruel de la femelle en partie sur la base de l'au moins une image de l'échantillon de sang ou de salive traité sur la bandelette de test immunologique (300) comprenant :
- la détermination (123) d'une mesure de l'estradiol dans l'échantillon de sang ou de salive et d'une mesure de l'hormone lutéinisante dans l'échantillon de sang ou de salive sur la base de l'au moins une image de l'échantillon de sang ou de salive ;
- le calcul (124) de l' instant t dans le temps pour la procédure médicale de fertilité dans le cycle menstruel de la femelle, en partie sur la base de la mesure de l'estradiol dans l'échantillon de sang ou de salive et de la mesure de l'hormone lutéinisante dans l'échantillon de sang ou de salive.

9. Procédé (100) selon l'une des revendications précédentes, lorsqu'elles dépendent de la revendication 6, le premier algorithme d'apprentissage automatique pré-entraîné ayant été entraîné en partie sur la base d'ensembles prédéterminés de données provenant d'autres femelles, éventuellement, chacun des ensembles prédéterminés de données comprenant au moins une autre image d'un échantillon de sang ou de salive d'une autre femelle traité sur une bandelette de test immunologique, un intervalle de temps pour une autre procédure médicale de fertilité indiquant une différence temporelle entre l'au moins une autre image et l'autre procédure médicale de fertilité, et un statut indiquant si l'autre procédure médicale de fertilité a abouti ou non à une grossesse de l'autre femelle et/ou à une naissance vivante.

10. Procédé (100) selon l'une des revendications précédentes, l'enregistrement des données comprenant en outre :
- l'enregistrement (143), sous l'identifiant unique, de l' instant t dans le temps pour la procédure médicale de fertilité ; et
- éventuellement, l'enregistrement (141), sous l'identifiant unique, du ou des éléments de métadonnées ; et
- éventuellement, l'obtention (144a) et l'enregistrement (144b), sous l'identifiant unique, d'un instant t dans le temps réel pour la procédure médicale de fertilité ; et
- éventuellement, l'obtention (147a) et l'enregistrement (147b), sous l'identifiant unique, d'un statut de grossesse de la femelle ; et
- éventuellement, l'obtention (148a) et l'enregistrement (148b), sous l'identifiant unique, d'un état de naissance vivante.

11. Procédé (100) selon l'une des revendications précédentes, lorsqu'elles dépendent de la revendication 9, comprenant en outre :
- l'ajout (153) aux ensembles prédéterminés de données provenant d'autres femelles, en tant que nouvel ensemble de données, des données enregistrées.

12. Procédé (100) selon l'une des revendications précédentes, l' instant t dans le temps calculé (120) pour la procédure médicale de fertilité étant :
- un horodatage spécifiant un jour du calendrier et/ou une heure du jour et/ou une minute de l'heure dans un fuseau horaire approprié ; ou
- un jour du calendrier, spécifiant éventuellement des intervalles de temps tels que matin, après-midi, soir et/ou nuit.

13. Serveur (160) dans un réseau, le serveur (160) étant configuré pour exécuter le procédé (100) pour calculer un instant t dans le temps pour une procédure médicale de fertilité dans un cycle menstruel d'une femelle selon l'une des revendications précédentes, éventuellement :
- l'au moins une image de l'échantillon de sang ou de salive traité sur la bandelette de test immunologique (300) ; et
- éventuellement, le ou les éléments de métadonnées ; et
- éventuellement, l'état de grossesse de la femelle ; et
- éventuellement, le statut de naissance vivante
étant obtenus par l'intermédiaire d'un ordinateur client (220) d'un kit d'analyse portable (200), et le serveur (160) fournissant l' instant t dans le temps pour la procédure médicale de fertilité dans le cycle menstruel de la femelle à l'ordinateur client (220) du kit d'analyse portable (200).

14. Kit d'analyse portable (200), comprenant :
- une bandelette de test immunologique (300) configurée pour traiter un échantillon de sang ou de salive ; et
- au moins un capteur d'image (210) configuré pour prendre, et éventuellement horodater, au moins une image de l'échantillon de sang ou de salive traité sur la bandelette de test immunologique (300) ; et
- un ordinateur client (220) configuré pour fournir un instant t dans le temps pour une procédure médicale de fertilité dans un cycle menstruel d'une femelle calculé sur la base de l'au moins une image de l'échantillon de sang ou de salive traité sur la bandelette de test immunologique (300) ;
l'instant t dans le temps pour la procédure médicale de fertilité dans le cycle menstruel de la femelle étant calculé conformément au procédé selon l'une des revendications 1 à 12, éventuellement, l'ordinateur client (220) étant en outre configuré pour communiquer avec un serveur (160) dans un réseau selon la revendication 13, et éventuellement, l'instant t dans le temps pour la procédure médicale de fertilité dans le cycle menstruel de la femelle étant calculé sur le serveur (160) conformément au procédé selon l'une des revendications 1 à 12.

15. Kit d'analyse portable (200) selon la revendication 14, comprenant en outre un dispositif d'analyse portable (230), le dispositif d'analyse portable (230) comprenant un support (231) pour maintenir la bandelette de test immunologique (300) configurée pour traiter l'échantillon de sang ou de salive, éventuellement, le dispositif d'analyse portable (230) comprenant en outre l'au moins un capteur d'image (210).
